# EUROPEAN PATENT APPLICATION

(11) **EP 3 733 697 A1**
(43) Date of publication of application: **04.11.2020**
(21) Application number: 19172048.1
(22) Date of filing: 30.04.2019
(51) Int. Cl.: C07K 14/725, C07K 14/705, C07K 14/74

(54) **ARTIFICIAL SIGNALLING MOLECULE**

(71) Applicant: Medizinische Hochschule Hannover, 30625 Hannover (DE)
(72) Inventor: Blasczyk, Rainer, 30916 Isernhagen (DE); Eiz-Vesper, Britta, 30625 Hannover (DE); Ferreira de Figueiredo, Constanca, 31535 Neustadt am Rübenberge (DE); Dragon, Anna, 30625 Hannover (DE)
(74) Representative: Taruttis, Stefan Georg

(57) **Abstract**

The invention provides a signalling molecule and an immune cell expressing the signalling molecule for use in the treatment of an undesired immune activity, which signalling molecule is a fusion protein which comprises a ligand domain, a spacer, a transmembrane domain, and at least one intracellular signalling domain, wherein the ligand domain comprises at least one epitope or all of the epitopes of the cognate antigen, or the cognate antigen, which cognate antigen is the target of the undesired immune activity.

SEQUENCE LISTING

<110> Medizinische Hochschule Hannover

<120> Artificial signalling molecule

<130> M1084EP

<160> 21

<170> BiSSAP 1.3.6

<210> 1
<211> 298
<212> PRT
<213> Artificial Sequence

<220>
<221> CHAIN
<222> 1..24
<223> signal peptide

<220>
<223> antigen domain HLA-A*02:01

<220>
<221> CHAIN
<222> 25..114
<223> alpha 1 domain

<220>
<221> CHAIN
<222> 115..206
<223> alpha 2 domain

<220>
<221> CHAIN
<222> 207..298
<223> alpha 3 domain

<400> 1
Met Ala Val Met Ala Pro Arg Thr Leu Val Leu Leu Leu Ser Gly Ala
1               5                   10                  15
Leu Ala Leu Thr Gln Thr Trp Ala Gly Ser His Ser Met Arg Tyr Phe
            20                  25                  30
Phe Thr Ser Val Ser Arg Pro Gly Arg Gly Glu Pro Arg Phe Ile Ala
        35                  40                  45
Val Gly Tyr Val Asp Asp Thr Gln Phe Val Arg Phe Asp Ser Asp Ala
    50                  55                  60
Ala Ser Gln Arg Met Glu Pro Arg Ala Pro Trp Ile Glu Gln Glu Gly
65                  70                  75                  80
Pro Glu Tyr Trp Asp Gly Glu Thr Arg Lys Val Lys Ala His Ser Gln
                85                  90                  95
Thr His Arg Val Asp Leu Gly Thr Leu Arg Gly Tyr Tyr Asn Gln Ser
            100                 105                 110
Glu Ala Gly Ser His Thr Val Gln Arg Met Tyr Gly Cys Asp Val Gly
        115                 120                 125
Ser Asp Trp Arg Phe Leu Arg Gly Tyr His Gln Tyr Ala Tyr Asp Gly
    130                 135                 140
Lys Asp Tyr Ile Ala Leu Lys Glu Asp Leu Arg Ser Trp Thr Ala Ala
145                 150                 155                 160
Asp Met Ala Ala Gln Thr Thr Lys His Lys Trp Glu Ala Ala His Val

                165                     170                     175
Ala Glu Gln Leu Arg Ala Tyr Leu Glu Gly Thr Cys Val Glu Trp Leu
            180                     185                     190
Arg Arg Tyr Leu Glu Asn Gly Lys Glu Thr Leu Gln Arg Thr Asp Ala
        195                     200                     205
Pro Lys Thr His Met Thr His His Ala Val Ser Asp His Glu Ala Thr
    210                     215                     220
Leu Arg Cys Trp Ala Leu Ser Phe Tyr Pro Ala Glu Ile Thr Leu Thr
225                     230                     235                     240
Trp Gln Arg Asp Gly Glu Asp Gln Thr Gln Asp Thr Glu Leu Val Glu
                245                     250                     255
Thr Arg Pro Ala Gly Asp Gly Thr Phe Gln Lys Trp Ala Ala Val Val
            260                     265                     270
Val Pro Ser Gly Gln Glu Gln Arg Tyr Thr Cys His Val Gln His Glu
        275                     280                     285
Gly Leu Pro Lys Pro Leu Thr Leu Arg Trp
    290                     295

<210> 2
<211> 257
<212> PRT
<213> Artificial Sequence

<220>
<221> CHAIN
<222> 1..25
<223> signal peptide

<220>
<223> fusion protein

<220>
<221> CHAIN
<222> 26..109
<223> alpha 1 domain

<220>
<221> CHAIN
<222> 110..203
<223> alpha 2 domain

<220>
<221> CHAIN
<222> 204..216
<223> extracellular portion of transmembrane domain

<220>
<221> CHAIN
<222> 217..223
<223> linker

<220>
<221> CHAIN
<222> 224..257
<223> Jun zipper

<400> 2
Met Ala Ile Ser Gly Val Pro Val Leu Gly Phe Phe Ile Ile Ala Val
1               5                   10                  15
Leu Met Ser Ala Gln Glu Ser Trp Ala Ile Lys Glu Glu His Val Ile
            20                  25                  30
Ile Gln Ala Glu Phe Tyr Leu Asn Pro Asp Gln Ser Gly Glu Phe Met

35                      40                      45
Phe Asp Phe Asp Gly Asp Glu Ile Phe His Val Asp Met Ala Lys Lys
    50                      55                      60
Glu Thr Val Trp Arg Leu Glu Glu Phe Gly Arg Phe Ala Ser Phe Glu
65                      70                      75                      80
Ala Gln Gly Ala Leu Ala Asn Ile Ala Val Asp Lys Ala Asn Leu Glu
                85                      90                      95
Ile Met Thr Lys Arg Ser Asn Tyr Thr Pro Ile Thr Asn Val Pro Pro
            100                     105                     110
Glu Val Thr Val Leu Thr Asn Ser Pro Val Glu Leu Arg Glu Pro Asn
        115                     120                     125
Val Leu Ile Cys Phe Ile Asp Lys Phe Thr Pro Pro Val Val Asn Val
    130                     135                     140
Thr Trp Leu Arg Asn Gly Lys Pro Val Thr Thr Gly Val Ser Glu Thr
145                     150                     155                     160
Val Phe Leu Pro Arg Glu Asp His Leu Phe Arg Lys Phe His Tyr Leu
                165                     170                     175
Pro Phe Leu Pro Ser Thr Glu Asp Val Tyr Asp Cys Arg Val Glu His
            180                     185                     190
Trp Gly Leu Asp Glu Pro Leu Leu Lys His Trp Glu Phe Asp Ala Pro
        195                     200                     205
Ser Pro Leu Pro Glu Thr Thr Glu Ser Arg Gly Gly Gly Gly Gly Ala
    210                     215                     220
Arg Leu Glu Glu Lys Val Lys Thr Leu Lys Ala Gln Asn Ser Glu Leu
225                     230                     235                     240
Ala Ser Thr Ala Asn Met Leu Arg Glu Gln Val Ala Gln Leu Lys Gln
                245                     250                     255
Ser


<210> 3
<211> 267
<212> PRT
<213> Artificial Sequence


<220>
<221> CHAIN
<222> 1..29
<223> signal peptide

<220>
<223> fusion protein

<220>
<221> CHAIN
<222> 30..123
<223> beta 1 chain

<220>
<221> CHAIN
<222> 124..217
<223> beta 2 chain

<220>
<221> CHAIN
<222> 218..227
<223> extracellular part of transmembrane domain

<220>
<221> CHAIN
<222> 228..234
<223> linker

<220>
<221> CHAIN
<222> 235..267
<223> Fos zipper

<400> 3
Met Val Cys Leu Lys Leu Pro Gly Gly Ser Cys Met Thr Ala Leu Thr
1               5                   10                  15
Val Thr Leu Met Val Leu Ser Ser Pro Leu Ala Leu Ala Gly Asp Thr
            20                  25                  30
Arg Pro Arg Phe Leu Trp Gln Leu Lys Phe Glu Cys His Phe Phe Asn
        35                  40                  45
Gly Thr Glu Arg Val Arg Leu Leu Glu Arg Cys Ile Tyr Asn Gln Glu
    50                  55                  60
Glu Ser Val Arg Phe Asp Ser Asp Val Gly Glu Tyr Arg Ala Val Thr
65                  70                  75                  80
Glu Leu Gly Arg Pro Asp Ala Glu Tyr Trp Asn Ser Gln Lys Asp Leu
                85                  90                  95
Leu Glu Gln Arg Arg Ala Ala Val Asp Thr Tyr Cys Arg His Asn Tyr
            100                 105                 110
Gly Val Gly Glu Ser Phe Thr Val Gln Arg Arg Val Glu Pro Lys Val
        115                 120                 125
Thr Val Tyr Pro Ser Lys Thr Gln Pro Leu Gln His His Asn Leu Leu
    130                 135                 140
Val Cys Ser Val Ser Gly Phe Tyr Pro Gly Ser Ile Glu Val Arg Trp
145                 150                 155                 160
Phe Arg Asn Gly Gln Glu Glu Lys Ala Gly Val Val Ser Thr Gly Leu
                165                 170                 175
Ile Gln Asn Gly Asp Trp Thr Phe Gln Thr Leu Val Met Leu Glu Thr
            180                 185                 190
Val Pro Arg Ser Gly Glu Val Tyr Thr Cys Gln Val Glu His Pro Ser
        195                 200                 205
Val Thr Ser Pro Leu Thr Val Glu Trp Arg Ala Arg Ser Glu Ser Ala
    210                 215                 220
Gln Ser Lys Ser Arg Gly Gly Gly Gly Gly Asp Thr Leu Gln Ala Glu
225                 230                 235                 240
Thr Asp Gln Leu Glu Asp Glu Lys Ser Ala Leu Gln Thr Glu Ile Ala
                245                 250                 255
Asn Leu Leu Lys Glu Lys Glu Lys Leu Glu Phe
            260                 265

<210> 4
<211> 34
<212> PRT
<213> Artificial Sequence

<220>
<221> CHAIN
<222> 1..4
<223> linker

<220>
<223> linker domain-acidic zipper domain

<220>
<221> CHAIN
<222> 5..34
<223> acidic zipper

<400> 4
Ser Arg Gly Thr Ala Gln Leu Glu Lys Glu Leu Gln Ala Leu Glu Lys

1                   5                   10                  15
Glu Asn Ala Gln Leu Glu Trp Glu Leu Gln Ala Leu Glu Lys Glu Leu
            20                  25                  30
Ala Gln



<210> 5
<211> 34
<212> PRT
<213> Artificial Sequence



<220>
<221> CHAIN
<222> 1..4
<223> linker

<220>
<223> linker domain-basic zipper domain

<220>
<221> CHAIN
<222> 5..34
<223> basic zipper

<400> 5
Ser Arg Gly Thr Ala Gln Leu Lys Lys Lys Leu Gln Ala Leu Lys Lys
1               5                   10                  15
Lys Asn Ala Gln Leu Lys Trp Lys Leu Gln Ala Leu Lys Lys Lys Leu
            20                  25                  30
Ala Gln



<210> 6
<211> 47
<212> PRT
<213> Artificial Sequence



<220>
<221> CHAIN
<222> 1..4
<223> linker

<220>
<223> linker domain-REL zipper domain

<220>
<221> CHAIN
<222> 5..47
<223> REL zipper

<400> 6
Ser Arg Gly Ser Leu Glu Ile Arg Ala Ala Phe Leu Arg Gln Arg Asn
1               5                   10                  15
Thr Ala Leu Arg Thr Glu Val Ala Glu Leu Glu Gln Glu Val Gln Arg
            20                  25                  30
Leu Glu Asn Glu Val Ser Gln Tyr Glu Thr Arg Tyr Gly Pro Leu
        35                  40                  45

<210> 7
<211> 47
<212> PRT

<213> Artificial Sequence

<220>
<221> CHAIN
<222> 1..4
<223> linker

<220>
<223> linker domain-ERL zipper domain

<220>
<221> CHAIN
<222> 5..47
<223> ERL zipper

<400> 7
Ser Arg Gly Thr Leu Glu Ile Glu Ala Ala Phe Leu Glu Arg Glu Asn
1               5                   10                  15
Thr Ala Leu Glu Thr Arg Val Ala Glu Leu Arg Gln Arg Val Gln Arg
            20                  25                  30
Leu Arg Asn Arg Val Ser Gln Tyr Arg Thr Arg Tyr Gly Pro Leu
        35                  40                  45

<210> 8
<211> 873
<212> PRT
<213> Artificial Sequence

<220>
<221> CHAIN
<222> 1..24
<223> signal peptide

<220>
<223> CLR with short spacer

<220>
<221> CHAIN
<222> 25..114
<223> alpha 1 domain

<220>
<221> CHAIN
<222> 115..206
<223> alpha 2 domain

<220>
<221> CHAIN
<222> 207..298
<223> alpha 3 domain

<220>
<221> CHAIN
<222> 299..310
<223> short spacer domain

<220>
<221> CHAIN
<222> 311..338
<223> CD28 transmembrane domain

<220>
<221> CHAIN
<222> 339..380
<223> 4-1BB costimulatory domain

<220>
<221> CHAIN
<222> 381..492
<223> CD3 zeta signalling domain

<220>
<221> CHAIN
<222> 493..516
<223> T2A element

<220>
<221> CHAIN
<222> 517..538
<223> GM-CSF signal peptide

<220>
<221> CHAIN
<222> 539..873
<223> truncated EGFR selection domain

<400> 8
Met Ala Val Met Ala Pro Arg Thr Leu Val Leu Leu Leu Ser Gly Ala
1               5                   10                  15
Leu Ala Leu Thr Gln Thr Trp Ala Gly Ser His Ser Met Arg Tyr Phe
            20                  25                  30
Phe Thr Ser Val Ser Arg Pro Gly Arg Gly Glu Pro Arg Phe Ile Ala
        35                  40                  45
Val Gly Tyr Val Asp Asp Thr Gln Phe Val Arg Phe Asp Ser Asp Ala
    50                  55                  60
Ala Ser Gln Arg Met Glu Pro Arg Ala Pro Trp Ile Glu Gln Glu Gly
65                  70                  75                  80
Pro Glu Tyr Trp Asp Gly Glu Thr Arg Lys Val Lys Ala His Ser Gln
                85                  90                  95
Thr His Arg Val Asp Leu Gly Thr Leu Arg Gly Tyr Tyr Asn Gln Ser
            100                 105                 110
Glu Ala Gly Ser His Thr Val Gln Arg Met Tyr Gly Cys Asp Val Gly
        115                 120                 125
Ser Asp Trp Arg Phe Leu Arg Gly Tyr His Gln Tyr Ala Tyr Asp Gly
    130                 135                 140
Lys Asp Tyr Ile Ala Leu Lys Glu Asp Leu Arg Ser Trp Thr Ala Ala
145                 150                 155                 160
Asp Met Ala Ala Gln Thr Thr Lys His Lys Trp Glu Ala Ala His Val
                165                 170                 175
Ala Glu Gln Leu Arg Ala Tyr Leu Glu Gly Thr Cys Val Glu Trp Leu
            180                 185                 190
Arg Arg Tyr Leu Glu Asn Gly Lys Glu Thr Leu Gln Arg Thr Asp Ala
        195                 200                 205
Pro Lys Thr His Met Thr His His Ala Val Ser Asp His Glu Ala Thr
    210                 215                 220
Leu Arg Cys Trp Ala Leu Ser Phe Tyr Pro Ala Glu Ile Thr Leu Thr
225                 230                 235                 240
Trp Gln Arg Asp Gly Glu Asp Gln Thr Gln Asp Thr Glu Leu Val Glu
                245                 250                 255
Thr Arg Pro Ala Gly Asp Gly Thr Phe Gln Lys Trp Ala Ala Val Val
            260                 265                 270
Val Pro Ser Gly Gln Glu Gln Arg Tyr Thr Cys His Val Gln His Glu
        275                 280                 285
Gly Leu Pro Lys Pro Leu Thr Leu Arg Trp Glu Ser Lys Tyr Gly Pro

290                 295                 300
Pro Cys Pro Pro Cys Pro Met Phe Trp Val Leu Val Val Val Gly Gly
305                 310                 315                 320
Val Leu Ala Cys Tyr Ser Leu Leu Val Thr Val Ala Phe Ile Ile Phe
                325                 330                 335
Trp Val Lys Arg Gly Arg Lys Lys Leu Leu Tyr Ile Phe Lys Gln Pro
            340                 345                 350
Phe Met Arg Pro Val Gln Thr Thr Gln Glu Glu Asp Gly Cys Ser Cys
        355                 360                 365
Arg Phe Pro Glu Glu Glu Glu Gly Gly Cys Glu Leu Arg Val Lys Phe
    370                 375                 380
Ser Arg Ser Ala Asp Ala Pro Ala Tyr Gln Gln Gly Gln Asn Gln Leu
385                 390                 395                 400
Tyr Asn Glu Leu Asn Leu Gly Arg Arg Glu Glu Tyr Asp Val Leu Asp
                405                 410                 415
Lys Arg Arg Gly Arg Asp Pro Glu Met Gly Gly Lys Pro Arg Arg Lys
            420                 425                 430
Asn Pro Gln Glu Gly Leu Tyr Asn Glu Leu Gln Lys Asp Lys Met Ala
        435                 440                 445
Glu Ala Tyr Ser Glu Ile Gly Met Lys Gly Glu Arg Arg Arg Gly Lys
    450                 455                 460
Gly His Asp Gly Leu Tyr Gln Gly Leu Ser Thr Ala Thr Lys Asp Thr
465                 470                 475                 480
Tyr Asp Ala Leu His Met Gln Ala Leu Pro Pro Arg Leu Glu Gly Gly
                485                 490                 495
Gly Glu Gly Arg Gly Ser Leu Leu Thr Cys Gly Asp Val Glu Glu Asn
            500                 505                 510
Pro Gly Pro Arg Met Leu Leu Leu Val Thr Ser Leu Leu Leu Cys Glu
        515                 520                 525
Leu Pro His Pro Ala Phe Leu Leu Ile Pro Arg Lys Val Cys Asn Gly
    530                 535                 540
Ile Gly Ile Gly Glu Phe Lys Asp Ser Leu Ser Ile Asn Ala Thr Asn
545                 550                 555                 560
Ile Lys His Phe Lys Asn Cys Thr Ser Ile Ser Gly Asp Leu His Ile
                565                 570                 575
Leu Pro Val Ala Phe Arg Gly Asp Ser Phe Thr His Thr Pro Pro Leu
            580                 585                 590
Asp Pro Gln Glu Leu Asp Ile Leu Lys Thr Val Lys Glu Ile Thr Gly
        595                 600                 605
Phe Leu Leu Ile Gln Ala Trp Pro Glu Asn Arg Thr Asp Leu His Ala
    610                 615                 620
Phe Glu Asn Leu Glu Ile Ile Arg Gly Arg Thr Lys Gln His Gly Gln
625                 630                 635                 640
Phe Ser Leu Ala Val Val Ser Leu Asn Ile Thr Ser Leu Gly Leu Arg
                645                 650                 655
Ser Leu Lys Glu Ile Ser Asp Gly Asp Val Ile Ile Ser Gly Asn Lys
            660                 665                 670
Asn Leu Cys Tyr Ala Asn Thr Ile Asn Trp Lys Lys Leu Phe Gly Thr
        675                 680                 685
Ser Gly Gln Lys Thr Lys Ile Ile Ser Asn Arg Gly Glu Asn Ser Cys
    690                 695                 700
Lys Ala Thr Gly Gln Val Cys His Ala Leu Cys Ser Pro Glu Gly Cys
705                 710                 715                 720
Trp Gly Pro Glu Pro Arg Asp Cys Val Ser Cys Arg Asn Val Ser Arg
                725                 730                 735
Gly Arg Glu Cys Val Asp Lys Cys Asn Leu Leu Glu Gly Glu Pro Arg
            740                 745                 750
Glu Phe Val Glu Asn Ser Glu Cys Ile Gln Cys His Pro Glu Cys Leu
        755                 760                 765
Pro Gln Ala Met Asn Ile Thr Cys Thr Gly Arg Gly Pro Asp Asn Cys
    770                 775                 780
Ile Gln Cys Ala His Tyr Ile Asp Gly Pro His Cys Val Lys Thr Cys
785                 790                 795                 800

Pro Ala Gly Val Met Gly Glu Asn Asn Thr Leu Val Trp Lys Tyr Ala
                805                 810                 815
Asp Ala Gly His Val Cys His Leu Cys His Pro Asn Cys Thr Tyr Gly
            820                 825                 830
Cys Thr Gly Pro Gly Leu Glu Gly Cys Pro Thr Asn Gly Pro Lys Ile
        835                 840                 845
Pro Ser Ile Ala Thr Gly Met Val Gly Ala Leu Leu Leu Leu Leu Val
    850                 855                 860
Val Ala Leu Gly Ile Gly Leu Phe Met
865                 870

<210> 9
<211> 1089
<212> PRT
<213> Artificial Sequence

<220>
<221> CHAIN
<222> 1..24
<223> signal peptide

<220>
<223> CLR long spacer construct

<220>
<221> CHAIN
<222> 25..114
<223> alpha 1 domain

<220>
<221> CHAIN
<222> 115..206
<223> alpha 2 domain

<220>
<221> CHAIN
<222> 207..298
<223> alpha 3 domain

<220>
<221> CHAIN
<222> 299..526
<223> long spacer domain

<220>
<221> CHAIN
<222> 527..554
<223> CD28 transmembrane domain

<220>
<221> CHAIN
<222> 555..596
<223> 4-1BB costimulatory domain

<220>
<221> CHAIN
<222> 597..708
<223> CD3 zeta signalling domain

<220>
<221> CHAIN
<222> 709..732

<223> T2A element

<220>
<221> CHAIN
<222> 734..754
<223> GM-CSF signal peptide

<220>
<221> CHAIN
<222> 755..1089
<223> truncated EGFR selection domain

<400> 9
Met Ala Val Met Ala Pro Arg Thr Leu Val Leu Leu Leu Ser Gly Ala
1               5                   10                  15
Leu Ala Leu Thr Gln Thr Trp Ala Gly Ser His Ser Met Arg Tyr Phe
            20                  25                  30
Phe Thr Ser Val Ser Arg Pro Gly Arg Gly Glu Pro Arg Phe Ile Ala
        35                  40                  45
Val Gly Tyr Val Asp Asp Thr Gln Phe Val Arg Phe Asp Ser Asp Ala
    50                  55                  60
Ala Ser Gln Arg Met Glu Pro Arg Ala Pro Trp Ile Glu Gln Glu Gly
65                  70                  75                  80
Pro Glu Tyr Trp Asp Gly Glu Thr Arg Lys Val Lys Ala His Ser Gln
                85                  90                  95
Thr His Arg Val Asp Leu Gly Thr Leu Arg Gly Tyr Tyr Asn Gln Ser
            100                 105                 110
Glu Ala Gly Ser His Thr Val Gln Arg Met Tyr Gly Cys Asp Val Gly
        115                 120                 125
Ser Asp Trp Arg Phe Leu Arg Gly Tyr His Gln Tyr Ala Tyr Asp Gly
    130                 135                 140
Lys Asp Tyr Ile Ala Leu Lys Glu Asp Leu Arg Ser Trp Thr Ala Ala
145                 150                 155                 160
Asp Met Ala Ala Gln Thr Thr Lys His Lys Trp Glu Ala Ala His Val
                165                 170                 175
Ala Glu Gln Leu Arg Ala Tyr Leu Glu Gly Thr Cys Val Glu Trp Leu
            180                 185                 190
Arg Arg Tyr Leu Glu Asn Gly Lys Glu Thr Leu Gln Arg Thr Asp Ala
        195                 200                 205
Pro Lys Thr His Met Thr His His Ala Val Ser Asp His Glu Ala Thr
    210                 215                 220
Leu Arg Cys Trp Ala Leu Ser Phe Tyr Pro Ala Glu Ile Thr Leu Thr
225                 230                 235                 240
Trp Gln Arg Asp Gly Glu Asp Gln Thr Gln Asp Thr Glu Leu Val Glu
                245                 250                 255
Thr Arg Pro Ala Gly Asp Gly Thr Phe Gln Lys Trp Ala Ala Val Val
            260                 265                 270
Val Pro Ser Gly Gln Glu Gln Arg Tyr Thr Cys His Val Gln His Glu
        275                 280                 285
Gly Leu Pro Lys Pro Leu Thr Leu Arg Trp Glu Ser Lys Tyr Gly Pro
    290                 295                 300
Pro Cys Pro Pro Cys Pro Ala Pro Pro Val Ala Gly Pro Ser Val Phe
305                 310                 315                 320
Leu Phe Pro Pro Lys Pro Lys Asp Thr Leu Met Ile Ser Arg Thr Pro
                325                 330                 335
Glu Val Thr Cys Val Val Val Asp Val Ser Gln Glu Asp Pro Glu Val
            340                 345                 350
Gln Phe Asn Trp Tyr Val Asp Gly Val Glu Val His Asn Ala Lys Thr
        355                 360                 365
Lys Pro Arg Glu Glu Gln Phe Gln Ser Thr Tyr Arg Val Val Ser Val
    370                 375                 380
Leu Thr Val Leu His Gln Asp Trp Leu Asn Gly Lys Glu Tyr Lys Cys
385                 390                 395                 400

Lys Val Ser Asn Lys Gly Leu Pro Ser Ser Ile Glu Lys Thr Ile Ser
                405                 410                 415
Lys Ala Lys Gly Gln Pro Arg Glu Pro Gln Val Tyr Thr Leu Pro Pro
            420                 425                 430
Ser Gln Glu Glu Met Thr Lys Asn Gln Val Ser Leu Thr Cys Leu Val
        435                 440                 445
Lys Gly Phe Tyr Pro Ser Asp Ile Ala Val Glu Trp Glu Ser Asn Gly
    450                 455                 460
Gln Pro Glu Asn Asn Tyr Lys Thr Thr Pro Pro Val Leu Asp Ser Asp
465                 470                 475                 480
Gly Ser Phe Phe Leu Tyr Ser Arg Leu Thr Val Asp Lys Ser Arg Trp
                485                 490                 495
Gln Glu Gly Asn Val Phe Ser Cys Ser Val Met His Glu Ala Leu His
            500                 505                 510
Asn His Tyr Thr Gln Lys Ser Leu Ser Leu Ser Leu Gly Lys Met Phe
        515                 520                 525
Trp Val Leu Val Val Val Gly Gly Val Leu Ala Cys Tyr Ser Leu Leu
    530                 535                 540
Val Thr Val Ala Phe Ile Ile Phe Trp Val Lys Arg Gly Arg Lys Lys
545                 550                 555                 560
Leu Leu Tyr Ile Phe Lys Gln Pro Phe Met Arg Pro Val Gln Thr Thr
                565                 570                 575
Gln Glu Glu Asp Gly Cys Ser Cys Arg Phe Pro Glu Glu Glu Glu Gly
            580                 585                 590
Gly Cys Glu Leu Arg Val Lys Phe Ser Arg Ser Ala Asp Ala Pro Ala
        595                 600                 605
Tyr Gln Gln Gly Gln Asn Gln Leu Tyr Asn Glu Leu Asn Leu Gly Arg
    610                 615                 620
Arg Glu Glu Tyr Asp Val Leu Asp Lys Arg Arg Gly Arg Asp Pro Glu
625                 630                 635                 640
Met Gly Gly Lys Pro Arg Arg Lys Asn Pro Gln Glu Gly Leu Tyr Asn
                645                 650                 655
Glu Leu Gln Lys Asp Lys Met Ala Glu Ala Tyr Ser Glu Ile Gly Met
            660                 665                 670
Lys Gly Glu Arg Arg Arg Gly Lys Gly His Asp Gly Leu Tyr Gln Gly
        675                 680                 685
Leu Ser Thr Ala Thr Lys Asp Thr Tyr Asp Ala Leu His Met Gln Ala
    690                 695                 700
Leu Pro Pro Arg Leu Glu Gly Gly Gly Glu Gly Arg Gly Ser Leu Leu
705                 710                 715                 720
Thr Cys Gly Asp Val Glu Glu Asn Pro Gly Pro Arg Met Leu Leu Leu
                725                 730                 735
Val Thr Ser Leu Leu Leu Cys Glu Leu Pro His Pro Ala Phe Leu Leu
            740                 745                 750
Ile Pro Arg Lys Val Cys Asn Gly Ile Gly Ile Gly Glu Phe Lys Asp
        755                 760                 765
Ser Leu Ser Ile Asn Ala Thr Asn Ile Lys His Phe Lys Asn Cys Thr
    770                 775                 780
Ser Ile Ser Gly Asp Leu His Ile Leu Pro Val Ala Phe Arg Gly Asp
785                 790                 795                 800
Ser Phe Thr His Thr Pro Pro Leu Asp Pro Gln Glu Leu Asp Ile Leu
                805                 810                 815
Lys Thr Val Lys Glu Ile Thr Gly Phe Leu Leu Ile Gln Ala Trp Pro
            820                 825                 830
Glu Asn Arg Thr Asp Leu His Ala Phe Glu Asn Leu Glu Ile Ile Arg
        835                 840                 845
Gly Arg Thr Lys Gln His Gly Gln Phe Ser Leu Ala Val Val Ser Leu
    850                 855                 860
Asn Ile Thr Ser Leu Gly Leu Arg Ser Leu Lys Glu Ile Ser Asp Gly
865                 870                 875                 880
Asp Val Ile Ile Ser Gly Asn Lys Asn Leu Cys Tyr Ala Asn Thr Ile
                885                 890                 895
Asn Trp Lys Lys Leu Phe Gly Thr Ser Gly Gln Lys Thr Lys Ile Ile

900                 905                 910
Ser Asn Arg Gly Glu Asn Ser Cys Lys Ala Thr Gly Gln Val Cys His
        915                 920                 925
Ala Leu Cys Ser Pro Glu Gly Cys Trp Gly Pro Glu Pro Arg Asp Cys
    930                 935                 940
Val Ser Cys Arg Asn Val Ser Arg Gly Arg Glu Cys Val Asp Lys Cys
945                 950                 955                 960
Asn Leu Leu Glu Gly Glu Pro Arg Glu Phe Val Glu Asn Ser Glu Cys
                965                 970                 975
Ile Gln Cys His Pro Glu Cys Leu Pro Gln Ala Met Asn Ile Thr Cys
            980                 985                 990
Thr Gly Arg Gly Pro Asp Asn Cys Ile Gln Cys Ala His Tyr Ile Asp
        995                 1000                1005
Gly Pro His Cys Val Lys Thr Cys Pro Ala Gly Val Met Gly Glu Asn
    1010                1015                1020
Asn Thr Leu Val Trp Lys Tyr Ala Asp Ala Gly His Val Cys His Leu
1025                1030                1035                1040
Cys His Pro Asn Cys Thr Tyr Gly Cys Thr Gly Pro Gly Leu Glu Gly
                1045                1050                1055
Cys Pro Thr Asn Gly Pro Lys Ile Pro Ser Ile Ala Thr Gly Met Val
            1060                1065                1070
Gly Ala Leu Leu Leu Leu Leu Val Val Ala Leu Gly Ile Gly Leu Phe
        1075                1080                1085
Met

<210> 10
<211> 832
<212> PRT
<213> Artificial Sequence

<220>
<221> CHAIN
<222> 1..25
<223> signal peptide

<220>
<223> HLA-DRA*01:01 CLR construct with Jun-zipper

<220>
<221> CHAIN
<222> 26..109
<223> alpha 1 domain

<220>
<221> CHAIN
<222> 110..203
<223> alpha 2 domain

<220>
<221> CHAIN
<222> 204..216
<223> extracellular part of transmembrane domain

<220>
<221> CHAIN
<222> 217..223
<223> linker

<220>
<221> CHAIN
<222> 224..257

<223> Jun-Zipper

<220>
<221> CHAIN
<222> 258..269
<223> short spacer domain

<220>
<221> CHAIN
<222> 270..297
<223> CD28 transmembrane domain

<220>
<221> CHAIN
<222> 298..339
<223> 4-1BB costimulatory domain

<220>
<221> CHAIN
<222> 340..451
<223> CD32 zeta signalling domain

<220>
<221> CHAIN
<222> 452..475
<223> T2A element

<220>
<221> CHAIN
<222> 476..497
<223> GM-CSF signal peptide

<220>
<221> CHAIN
<222> 498..832
<223> truncated EGFR selection domain

<400> 10
Met Ala Ile Ser Gly Val Pro Val Leu Gly Phe Phe Ile Ile Ala Val
1               5                   10                  15
Leu Met Ser Ala Gln Glu Ser Trp Ala Ile Lys Glu Glu His Val Ile
            20                  25                  30
Ile Gln Ala Glu Phe Tyr Leu Asn Pro Asp Gln Ser Gly Glu Phe Met
        35                  40                  45
Phe Asp Phe Asp Gly Asp Glu Ile Phe His Val Asp Met Ala Lys Lys
    50                  55                  60
Glu Thr Val Trp Arg Leu Glu Glu Phe Gly Arg Phe Ala Ser Phe Glu
65                  70                  75                  80
Ala Gln Gly Ala Leu Ala Asn Ile Ala Val Asp Lys Ala Asn Leu Glu
                85                  90                  95
Ile Met Thr Lys Arg Ser Asn Tyr Thr Pro Ile Thr Asn Val Pro Pro
            100                 105                 110
Glu Val Thr Val Leu Thr Asn Ser Pro Val Glu Leu Arg Glu Pro Asn
        115                 120                 125
Val Leu Ile Cys Phe Ile Asp Lys Phe Thr Pro Pro Val Val Asn Val
    130                 135                 140
Thr Trp Leu Arg Asn Gly Lys Pro Val Thr Thr Gly Val Ser Glu Thr
145                 150                 155                 160
Val Phe Leu Pro Arg Glu Asp His Leu Phe Arg Lys Phe His Tyr Leu
                165                 170                 175
Pro Phe Leu Pro Ser Thr Glu Asp Val Tyr Asp Cys Arg Val Glu His
            180                 185                 190
Trp Gly Leu Asp Glu Pro Leu Leu Lys His Trp Glu Phe Asp Ala Pro

        195                 200                 205
Ser Pro Leu Pro Glu Thr Thr Glu Ser Arg Gly Gly Gly Gly Gly Ala
    210                 215                 220
Arg Leu Glu Glu Lys Val Lys Thr Leu Lys Ala Gln Asn Ser Glu Leu
225                 230                 235                 240
Ala Ser Thr Ala Asn Met Leu Arg Glu Gln Val Ala Gln Leu Lys Gln
                245                 250                 255
Ser Glu Ser Lys Tyr Gly Pro Pro Cys Pro Pro Cys Pro Met Phe Trp
            260                 265                 270
Val Leu Val Val Val Gly Gly Val Leu Ala Cys Tyr Ser Leu Leu Val
        275                 280                 285
Thr Val Ala Phe Ile Ile Phe Trp Val Lys Arg Gly Arg Lys Lys Leu
    290                 295                 300
Leu Tyr Ile Phe Lys Gln Pro Phe Met Arg Pro Val Gln Thr Thr Gln
305                 310                 315                 320
Glu Glu Asp Gly Cys Ser Cys Arg Phe Pro Glu Glu Glu Glu Gly Gly
                325                 330                 335
Cys Glu Leu Arg Val Lys Phe Ser Arg Ser Ala Asp Ala Pro Ala Tyr
            340                 345                 350
Gln Gln Gly Gln Asn Gln Leu Tyr Asn Glu Leu Asn Leu Gly Arg Arg
        355                 360                 365
Glu Glu Tyr Asp Val Leu Asp Lys Arg Arg Gly Arg Asp Pro Glu Met
    370                 375                 380
Gly Gly Lys Pro Arg Arg Lys Asn Pro Gln Glu Gly Leu Tyr Asn Glu
385                 390                 395                 400
Leu Gln Lys Asp Lys Met Ala Glu Ala Tyr Ser Glu Ile Gly Met Lys
                405                 410                 415
Gly Glu Arg Arg Arg Gly Lys Gly His Asp Gly Leu Tyr Gln Gly Leu
            420                 425                 430
Ser Thr Ala Thr Lys Asp Thr Tyr Asp Ala Leu His Met Gln Ala Leu
        435                 440                 445
Pro Pro Arg Leu Glu Gly Gly Gly Glu Gly Arg Gly Ser Leu Leu Thr
    450                 455                 460
Cys Gly Asp Val Glu Glu Asn Pro Gly Pro Arg Met Leu Leu Leu Val
465                 470                 475                 480
Thr Ser Leu Leu Leu Cys Glu Leu Pro His Pro Ala Phe Leu Leu Ile
                485                 490                 495
Pro Arg Lys Val Cys Asn Gly Ile Gly Ile Gly Glu Phe Lys Asp Ser
            500                 505                 510
Leu Ser Ile Asn Ala Thr Asn Ile Lys His Phe Lys Asn Cys Thr Ser
        515                 520                 525
Ile Ser Gly Asp Leu His Ile Leu Pro Val Ala Phe Arg Gly Asp Ser
    530                 535                 540
Phe Thr His Thr Pro Pro Leu Asp Pro Gln Glu Leu Asp Ile Leu Lys
545                 550                 555                 560
Thr Val Lys Glu Ile Thr Gly Phe Leu Leu Ile Gln Ala Trp Pro Glu
                565                 570                 575
Asn Arg Thr Asp Leu His Ala Phe Glu Asn Leu Glu Ile Ile Arg Gly
            580                 585                 590
Arg Thr Lys Gln His Gly Gln Phe Ser Leu Ala Val Val Ser Leu Asn
        595                 600                 605
Ile Thr Ser Leu Gly Leu Arg Ser Leu Lys Glu Ile Ser Asp Gly Asp
    610                 615                 620
Val Ile Ile Ser Gly Asn Lys Asn Leu Cys Tyr Ala Asn Thr Ile Asn
625                 630                 635                 640
Trp Lys Lys Leu Phe Gly Thr Ser Gly Gln Lys Thr Lys Ile Ile Ser
                645                 650                 655
Asn Arg Gly Glu Asn Ser Cys Lys Ala Thr Gly Gln Val Cys His Ala
            660                 665                 670
Leu Cys Ser Pro Glu Gly Cys Trp Gly Pro Glu Pro Arg Asp Cys Val
        675                 680                 685
Ser Cys Arg Asn Val Ser Arg Gly Arg Glu Cys Val Asp Lys Cys Asn
    690                 695                 700

Leu Leu Glu Gly Glu Pro Arg Glu Phe Val Glu Asn Ser Glu Cys Ile
705                 710                 715                 720
Gln Cys His Pro Glu Cys Leu Pro Gln Ala Met Asn Ile Thr Cys Thr
                725                 730                 735
Gly Arg Gly Pro Asp Asn Cys Ile Gln Cys Ala His Tyr Ile Asp Gly
            740                 745                 750
Pro His Cys Val Lys Thr Cys Pro Ala Gly Val Met Gly Glu Asn Asn
        755                 760                 765
Thr Leu Val Trp Lys Tyr Ala Asp Ala Gly His Val Cys His Leu Cys
    770                 775                 780
His Pro Asn Cys Thr Tyr Gly Cys Thr Gly Pro Gly Leu Glu Gly Cys
785                 790                 795                 800
Pro Thr Asn Gly Pro Lys Ile Pro Ser Ile Ala Thr Gly Met Val Gly
                805                 810                 815
Ala Leu Leu Leu Leu Leu Val Val Ala Leu Gly Ile Gly Leu Phe Met
            820                 825                 830

<210> 11
<211> 842
<212> PRT
<213> Artificial Sequence

<220>
<221> CHAIN
<222> 1..29
<223> signal peptide

<220>
<223> HLA-DRB1*01:01 CLR short construct with Fos-zipper

<220>
<221> CHAIN
<222> 30..123
<223> beta 1 domain

<220>
<221> CHAIN
<222> 124..217
<223> beta 2 domain

<220>
<221> CHAIN
<222> 218..227
<223> extracellular part of transmembrane domain

<220>
<221> CHAIN
<222> 228..234
<223> linker

<220>
<221> CHAIN
<222> 235..267
<223> Fos-Zipper

<220>
<221> CHAIN
<222> 268..279
<223> short hinge domain
      short spacer domain

<220>
<221> CHAIN
<222> 280..307
<223> CD28 transmembrane domain

<220>
<221> CHAIN
<222> 308..349
<223> 4-1BB costimulatory domain

<220>
<221> CHAIN
<222> 350..461
<223> CD3 zeta signalling domain

<220>
<221> CHAIN
<222> 462..485
<223> T2A element

<220>
<221> CHAIN
<222> 486..507
<223> GM-CSF signal peptide

<220>
<221> CHAIN
<222> 508..842
<223> truncated EGFR selection domain

<400> 11
Met Val Cys Leu Lys Leu Pro Gly Gly Ser Cys Met Thr Ala Leu Thr
1               5                   10                  15
Val Thr Leu Met Val Leu Ser Ser Pro Leu Ala Leu Ala Gly Asp Thr
            20                  25                  30
Arg Pro Arg Phe Leu Trp Gln Leu Lys Phe Glu Cys His Phe Phe Asn
        35                  40                  45
Gly Thr Glu Arg Val Arg Leu Leu Glu Arg Cys Ile Tyr Asn Gln Glu
    50                  55                  60
Glu Ser Val Arg Phe Asp Ser Asp Val Gly Glu Tyr Arg Ala Val Thr
65                  70                  75                  80
Glu Leu Gly Arg Pro Asp Ala Glu Tyr Trp Asn Ser Gln Lys Asp Leu
                85                  90                  95
Leu Glu Gln Arg Arg Ala Ala Val Asp Thr Tyr Cys Arg His Asn Tyr
            100                 105                 110
Gly Val Gly Glu Ser Phe Thr Val Gln Arg Arg Val Glu Pro Lys Val
        115                 120                 125
Thr Val Tyr Pro Ser Lys Thr Gln Pro Leu Gln His His Asn Leu Leu
    130                 135                 140
Val Cys Ser Val Ser Gly Phe Tyr Pro Gly Ser Ile Glu Val Arg Trp
145                 150                 155                 160
Phe Arg Asn Gly Gln Glu Glu Lys Ala Gly Val Val Ser Thr Gly Leu
                165                 170                 175
Ile Gln Asn Gly Asp Trp Thr Phe Gln Thr Leu Val Met Leu Glu Thr
            180                 185                 190
Val Pro Arg Ser Gly Glu Val Tyr Thr Cys Gln Val Glu His Pro Ser
        195                 200                 205
Val Thr Ser Pro Leu Thr Val Glu Trp Arg Ala Arg Ser Glu Ser Ala
    210                 215                 220
Gln Ser Lys Ser Arg Gly Gly Gly Gly Gly Asp Thr Leu Gln Ala Glu
225                 230                 235                 240
Thr Asp Gln Leu Glu Asp Glu Lys Ser Ala Leu Gln Thr Glu Ile Ala

                    245                 250                 255
Asn Leu Leu Lys Glu Lys Glu Lys Leu Glu Phe Glu Ser Lys Tyr Gly
            260                 265                 270
Pro Pro Cys Pro Pro Cys Pro Met Phe Trp Val Leu Val Val Val Gly
        275                 280                 285
Gly Val Leu Ala Cys Tyr Ser Leu Leu Val Thr Val Ala Phe Ile Ile
    290                 295                 300
Phe Trp Val Lys Arg Gly Arg Lys Lys Leu Leu Tyr Ile Phe Lys Gln
305                 310                 315                 320
Pro Phe Met Arg Pro Val Gln Thr Thr Gln Glu Glu Asp Gly Cys Ser
                325                 330                 335
Cys Arg Phe Pro Glu Glu Glu Glu Gly Gly Cys Glu Leu Arg Val Lys
            340                 345                 350
Phe Ser Arg Ser Ala Asp Ala Pro Ala Tyr Gln Gln Gly Gln Asn Gln
        355                 360                 365
Leu Tyr Asn Glu Leu Asn Leu Gly Arg Arg Glu Glu Tyr Asp Val Leu
    370                 375                 380
Asp Lys Arg Arg Gly Arg Asp Pro Glu Met Gly Gly Lys Pro Arg Arg
385                 390                 395                 400
Lys Asn Pro Gln Glu Gly Leu Tyr Asn Glu Leu Gln Lys Asp Lys Met
                405                 410                 415
Ala Glu Ala Tyr Ser Glu Ile Gly Met Lys Gly Glu Arg Arg Arg Gly
            420                 425                 430
Lys Gly His Asp Gly Leu Tyr Gln Gly Leu Ser Thr Ala Thr Lys Asp
        435                 440                 445
Thr Tyr Asp Ala Leu His Met Gln Ala Leu Pro Pro Arg Leu Glu Gly
    450                 455                 460
Gly Gly Glu Gly Arg Gly Ser Leu Leu Thr Cys Gly Asp Val Glu Glu
465                 470                 475                 480
Asn Pro Gly Pro Arg Met Leu Leu Leu Val Thr Ser Leu Leu Leu Cys
                485                 490                 495
Glu Leu Pro His Pro Ala Phe Leu Leu Ile Pro Arg Lys Val Cys Asn
            500                 505                 510
Gly Ile Gly Ile Gly Glu Phe Lys Asp Ser Leu Ser Ile Asn Ala Thr
        515                 520                 525
Asn Ile Lys His Phe Lys Asn Cys Thr Ser Ile Ser Gly Asp Leu His
    530                 535                 540
Ile Leu Pro Val Ala Phe Arg Gly Asp Ser Phe Thr His Thr Pro Pro
545                 550                 555                 560
Leu Asp Pro Gln Glu Leu Asp Ile Leu Lys Thr Val Lys Glu Ile Thr
                565                 570                 575
Gly Phe Leu Leu Ile Gln Ala Trp Pro Glu Asn Arg Thr Asp Leu His
            580                 585                 590
Ala Phe Glu Asn Leu Glu Ile Ile Arg Gly Arg Thr Lys Gln His Gly
        595                 600                 605
Gln Phe Ser Leu Ala Val Val Ser Leu Asn Ile Thr Ser Leu Gly Leu
    610                 615                 620
Arg Ser Leu Lys Glu Ile Ser Asp Gly Asp Val Ile Ile Ser Gly Asn
625                 630                 635                 640
Lys Asn Leu Cys Tyr Ala Asn Thr Ile Asn Trp Lys Lys Leu Phe Gly
                645                 650                 655
Thr Ser Gly Gln Lys Thr Lys Ile Ile Ser Asn Arg Gly Glu Asn Ser
            660                 665                 670
Cys Lys Ala Thr Gly Gln Val Cys His Ala Leu Cys Ser Pro Glu Gly
        675                 680                 685
Cys Trp Gly Pro Glu Pro Arg Asp Cys Val Ser Cys Arg Asn Val Ser
    690                 695                 700
Arg Gly Arg Glu Cys Val Asp Lys Cys Asn Leu Leu Glu Gly Glu Pro
705                 710                 715                 720
Arg Glu Phe Val Glu Asn Ser Glu Cys Ile Gln Cys His Pro Glu Cys
                725                 730                 735
Leu Pro Gln Ala Met Asn Ile Thr Cys Thr Gly Arg Gly Pro Asp Asn
            740                 745                 750

Cys Ile Gln Cys Ala His Tyr Ile Asp Gly Pro His Cys Val Lys Thr
        755                 760                 765
Cys Pro Ala Gly Val Met Gly Glu Asn Asn Thr Leu Val Trp Lys Tyr
    770                 775                 780
Ala Asp Ala Gly His Val Cys His Leu Cys His Pro Asn Cys Thr Tyr
785                 790                 795                 800
Gly Cys Thr Gly Pro Gly Leu Glu Gly Cys Pro Thr Asn Gly Pro Lys
                805                 810                 815
Ile Pro Ser Ile Ala Thr Gly Met Val Gly Ala Leu Leu Leu Leu Leu
            820                 825                 830
Val Val Ala Leu Gly Ile Gly Leu Phe Met
        835                 840

<210> 12
<211> 1048
<212> PRT
<213> Artificial Sequence

<220>
<221> CHAIN
<222> 1..25
<223> signal peptide

<220>
<223> HLA-DRA*01:01 CLR long construct with Jun-Zipper

<220>
<221> CHAIN
<222> 26..109
<223> alpha 1 domain

<220>
<221> CHAIN
<222> 110..203
<223> alpha 2 domain

<220>
<221> CHAIN
<222> 204..216
<223> extracellular part of transmembrane domain

<220>
<221> CHAIN
<222> 217..223
<223> linker

<220>
<221> CHAIN
<222> 224..257
<223> Jun-Zipper

<220>
<221> CHAIN
<222> 258..485
<223> long spacer domain

<220>
<221> CHAIN
<222> 486..513
<223> CD28 transmembrane domain

<220>

<221> CHAIN
<222> 514..555
<223> 4-1BB costimulatory domain

<220>
<221> CHAIN
<222> 556..667
<223> CD3 zeta signalling domain

<220>
<221> CHAIN
<222> 668..691
<223> T2A element

<220>
<221> CHAIN
<222> 692..713
<223> GM-CSF signal peptide

<220>
<221> CHAIN
<222> 714..1048
<223> truncated EGFR selection domain

<400> 12
   Met Ala Ile Ser Gly Val Pro Val Leu Gly Phe Phe Ile Ile Ala Val
   1               5                   10                  15
   Leu Met Ser Ala Gln Glu Ser Trp Ala Ile Lys Glu Glu His Val Ile
               20                  25                  30
   Ile Gln Ala Glu Phe Tyr Leu Asn Pro Asp Gln Ser Gly Glu Phe Met
           35                  40                  45
   Phe Asp Phe Asp Gly Asp Glu Ile Phe His Val Asp Met Ala Lys Lys
       50                  55                  60
   Glu Thr Val Trp Arg Leu Glu Glu Phe Gly Arg Phe Ala Ser Phe Glu
   65                  70                  75                  80
   Ala Gln Gly Ala Leu Ala Asn Ile Ala Val Asp Lys Ala Asn Leu Glu
                   85                  90                  95
   Ile Met Thr Lys Arg Ser Asn Tyr Thr Pro Ile Thr Asn Val Pro Pro
               100                 105                 110
   Glu Val Thr Val Leu Thr Asn Ser Pro Val Glu Leu Arg Glu Pro Asn
           115                 120                 125
   Val Leu Ile Cys Phe Ile Asp Lys Phe Thr Pro Pro Val Val Asn Val
       130                 135                 140
   Thr Trp Leu Arg Asn Gly Lys Pro Val Thr Thr Gly Val Ser Glu Thr
   145                 150                 155                 160
   Val Phe Leu Pro Arg Glu Asp His Leu Phe Arg Lys Phe His Tyr Leu
                   165                 170                 175
   Pro Phe Leu Pro Ser Thr Glu Asp Val Tyr Asp Cys Arg Val Glu His
               180                 185                 190
   Trp Gly Leu Asp Glu Pro Leu Leu Lys His Trp Glu Phe Asp Ala Pro
           195                 200                 205
   Ser Pro Leu Pro Glu Thr Thr Glu Ser Arg Gly Gly Gly Gly Gly Ala
       210                 215                 220
   Arg Leu Glu Glu Lys Val Lys Thr Leu Lys Ala Gln Asn Ser Glu Leu
   225                 230                 235                 240
   Ala Ser Thr Ala Asn Met Leu Arg Glu Gln Val Ala Gln Leu Lys Gln
                   245                 250                 255
   Ser Glu Ser Lys Tyr Gly Pro Pro Cys Pro Pro Cys Pro Ala Pro Pro
               260                 265                 270
   Val Ala Gly Pro Ser Val Phe Leu Phe Pro Pro Lys Pro Lys Asp Thr
           275                 280                 285
   Leu Met Ile Ser Arg Thr Pro Glu Val Thr Cys Val Val Val Asp Val
       290                 295                 300
   Ser Gln Glu Asp Pro Glu Val Gln Phe Asn Trp Tyr Val Asp Gly Val
   305                 310                 315                 320
   Glu Val His Asn Ala Lys Thr Lys Pro Arg Glu Glu Gln Phe Gln Ser
                   325                 330                 335
   Thr Tyr Arg Val Val Ser Val Leu Thr Val Leu His Gln Asp Trp Leu
               340                 345                 350
   Asn Gly Lys Glu Tyr Lys Cys Lys Val Ser Asn Lys Gly Leu Pro Ser
           355                 360                 365
   Ser Ile Glu Lys Thr Ile Ser Lys Ala Lys Gly Gln Pro Arg Glu Pro
       370                 375                 380
   Gln Val Tyr Thr Leu Pro Pro Ser Gln Glu Glu Met Thr Lys Asn Gln
   385                 390                 395                 400
   Val Ser Leu Thr Cys Leu Val Lys Gly Phe Tyr Pro Ser Asp Ile Ala
                   405                 410                 415
   Val Glu Trp Glu Ser Asn Gly Gln Pro Glu Asn Asn Tyr Lys Thr Thr
               420                 425                 430
   Pro Pro Val Leu Asp Ser Asp Gly Ser Phe Phe Leu Tyr Ser Arg Leu
           435                 440                 445
   Thr Val Asp Lys Ser Arg Trp Gln Glu Gly Asn Val Phe Ser Cys Ser
       450                 455                 460
   Val Met His Glu Ala Leu His Asn His Tyr Thr Gln Lys Ser Leu Ser
   465                 470                 475                 480
   Leu Ser Leu Gly Lys Met Phe Trp Val Leu Val Val Val Gly Gly Val
                   485                 490                 495
   Leu Ala Cys Tyr Ser Leu Leu Val Thr Val Ala Phe Ile Ile Phe Trp
               500                 505                 510
   Val Lys Arg Gly Arg Lys Lys Leu Leu Tyr Ile Phe Lys Gln Pro Phe
           515                 520                 525
   Met Arg Pro Val Gln Thr Thr Gln Glu Glu Asp Gly Cys Ser Cys Arg
       530                 535                 540
   Phe Pro Glu Glu Glu Glu Gly Gly Cys Glu Leu Arg Val Lys Phe Ser
   545                 550                 555                 560
   Arg Ser Ala Asp Ala Pro Ala Tyr Gln Gln Gly Gln Asn Gln Leu Tyr
                   565                 570                 575
   Asn Glu Leu Asn Leu Gly Arg Arg Glu Glu Tyr Asp Val Leu Asp Lys
               580                 585                 590
   Arg Arg Gly Arg Asp Pro Glu Met Gly Gly Lys Pro Arg Arg Lys Asn
           595                 600                 605
   Pro Gln Glu Gly Leu Tyr Asn Glu Leu Gln Lys Asp Lys Met Ala Glu
       610                 615                 620
   Ala Tyr Ser Glu Ile Gly Met Lys Gly Glu Arg Arg Arg Gly Lys Gly
   625                 630                 635                 640
   His Asp Gly Leu Tyr Gln Gly Leu Ser Thr Ala Thr Lys Asp Thr Tyr
                   645                 650                 655
   Asp Ala Leu His Met Gln Ala Leu Pro Pro Arg Leu Glu Gly Gly Gly
               660                 665                 670
   Glu Gly Arg Gly Ser Leu Leu Thr Cys Gly Asp Val Glu Glu Asn Pro
           675                 680                 685
   Gly Pro Arg Met Leu Leu Leu Val Thr Ser Leu Leu Leu Cys Glu Leu
       690                 695                 700
   Pro His Pro Ala Phe Leu Leu Ile Pro Arg Lys Val Cys Asn Gly Ile
   705                 710                 715                 720
   Gly Ile Gly Glu Phe Lys Asp Ser Leu Ser Ile Asn Ala Thr Asn Ile
                   725                 730                 735
   Lys His Phe Lys Asn Cys Thr Ser Ile Ser Gly Asp Leu His Ile Leu
               740                 745                 750
   Pro Val Ala Phe Arg Gly Asp Ser Phe Thr His Thr Pro Pro Leu Asp
           755                 760                 765
   Pro Gln Glu Leu Asp Ile Leu Lys Thr Val Lys Glu Ile Thr Gly Phe
       770                 775                 780
   Leu Leu Ile Gln Ala Trp Pro Glu Asn Arg Thr Asp Leu His Ala Phe
   785                 790                 795                 800
   Glu Asn Leu Glu Ile Ile Arg Gly Arg Thr Lys Gln His Gly Gln Phe
                    805                 810                 815
   Ser Leu Ala Val Val Ser Leu Asn Ile Thr Ser Leu Gly Leu Arg Ser
               820                 825                 830
   Leu Lys Glu Ile Ser Asp Gly Asp Val Ile Ile Ser Gly Asn Lys Asn
           835                 840                 845
   Leu Cys Tyr Ala Asn Thr Ile Asn Trp Lys Lys Leu Phe Gly Thr Ser
       850                 855                 860
   Gly Gln Lys Thr Lys Ile Ile Ser Asn Arg Gly Glu Asn Ser Cys Lys
   865                 870                 875                 880
   Ala Thr Gly Gln Val Cys His Ala Leu Cys Ser Pro Glu Gly Cys Trp
                   885                 890                 895
   Gly Pro Glu Pro Arg Asp Cys Val Ser Cys Arg Asn Val Ser Arg Gly
               900                 905                 910
   Arg Glu Cys Val Asp Lys Cys Asn Leu Leu Glu Gly Glu Pro Arg Glu
           915                 920                 925
   Phe Val Glu Asn Ser Glu Cys Ile Gln Cys His Pro Glu Cys Leu Pro
       930                 935                 940
   Gln Ala Met Asn Ile Thr Cys Thr Gly Arg Gly Pro Asp Asn Cys Ile
   945                 950                 955                 960
   Gln Cys Ala His Tyr Ile Asp Gly Pro His Cys Val Lys Thr Cys Pro
                   965                 970                 975
   Ala Gly Val Met Gly Glu Asn Asn Thr Leu Val Trp Lys Tyr Ala Asp
               980                 985                 990
   Ala Gly His Val Cys His Leu Cys His Pro Asn Cys Thr Tyr Gly Cys
           995                 1000                1005
   Thr Gly Pro Gly Leu Glu Gly Cys Pro Thr Asn Gly Pro Lys Ile Pro
       1010                1015                1020
   Ser Ile Ala Thr Gly Met Val Gly Ala Leu Leu Leu Leu Leu Val Val
   1025                1030                1035                1040
   Ala Leu Gly Ile Gly Leu Phe Met
                   1045

<210> 13
<211> 1058
<212> PRT
<213> Artificial Sequence



<220>
<221> CHAIN
<222> 1..29
<223> signal peptide

<220>
<223> HLA-DRB1*01:01 CLR long construct with Fos-Zipper

<220>
<221> CHAIN
<222> 30..123
<223> beta 1 domain

<220>
<221> CHAIN
<222> 124..217
<223> beta 2 domain

<220>
<221> CHAIN
<222> 218..227
<223> extracellular part of transmembrane domain

<220>
<221> CHAIN

<222> 228..234
<223> linker

<220>
<221> CHAIN
<222> 234..267
<223> Fos-Zipper

<220>
<221> CHAIN
<222> 268..495
<223> long spacer domain

<220>
<221> CHAIN
<222> 496..523
<223> CD28 transmembrane domain

<220>
<221> CHAIN
<222> 524..565
<223> 4-1BB costimulatory domain

<220>
<221> CHAIN
<222> 566..677
<223> CD3 zeta signalling domain

<220>
<221> CHAIN
<222> 678..701
<223> T2A element

<220>
<221> CHAIN
<222> 702..723
<223> GM-CSF signal peptide

<220>
<221> CHAIN
<222> 724..1058
<223> truncated EGFR selection domain

<400> 13
   Met Val Cys Leu Lys Leu Pro Gly Gly Ser Cys Met Thr Ala Leu Thr
   1               5                   10                  15
   Val Thr Leu Met Val Leu Ser Ser Pro Leu Ala Leu Ala Gly Asp Thr
               20                  25                  30
   Arg Pro Arg Phe Leu Trp Gln Leu Lys Phe Glu Cys His Phe Phe Asn
           35                  40                  45
   Gly Thr Glu Arg Val Arg Leu Leu Glu Arg Cys Ile Tyr Asn Gln Glu
       50                  55                  60
   Glu Ser Val Arg Phe Asp Ser Asp Val Gly Glu Tyr Arg Ala Val Thr
   65                  70                  75                  80
   Glu Leu Gly Arg Pro Asp Ala Glu Tyr Trp Asn Ser Gln Lys Asp Leu
                   85                  90                  95
   Leu Glu Gln Arg Arg Ala Ala Val Asp Thr Tyr Cys Arg His Asn Tyr
               100                 105                 110
   Gly Val Gly Glu Ser Phe Thr Val Gln Arg Arg Val Glu Pro Lys Val
           115                 120                 125
   Thr Val Tyr Pro Ser Lys Thr Gln Pro Leu Gln His His Asn Leu Leu
       130                 135                 140
   Val Cys Ser Val Ser Gly Phe Tyr Pro Gly Ser Ile Glu Val Arg Trp
   145                 150                 155                 160
   Phe Arg Asn Gly Gln Glu Glu Lys Ala Gly Val Val Ser Thr Gly Leu
                   165                 170                 175
   Ile Gln Asn Gly Asp Trp Thr Phe Gln Thr Leu Val Met Leu Glu Thr
               180                 185                 190
   Val Pro Arg Ser Gly Glu Val Tyr Thr Cys Gln Val Glu His Pro Ser
           195                 200                 205
   Val Thr Ser Pro Leu Thr Val Glu Trp Arg Ala Arg Ser Glu Ser Ala
       210                 215                 220
   Gln Ser Lys Ser Arg Gly Gly Gly Gly Gly Asp Thr Leu Gln Ala Glu
   225                 230                 235                 240
   Thr Asp Gln Leu Glu Asp Glu Lys Ser Ala Leu Gln Thr Glu Ile Ala
                   245                 250                 255
   Asn Leu Leu Lys Glu Lys Glu Lys Leu Glu Phe Glu Ser Lys Tyr Gly
               260                 265                 270
   Pro Pro Cys Pro Pro Cys Pro Ala Pro Pro Val Ala Gly Pro Ser Val
           275                 280                 285
   Phe Leu Phe Pro Pro Lys Pro Lys Asp Thr Leu Met Ile Ser Arg Thr
       290                 295                 300
   Pro Glu Val Thr Cys Val Val Val Asp Val Ser Gln Glu Asp Pro Glu
   305                 310                 315                 320
   Val Gln Phe Asn Trp Tyr Val Asp Gly Val Glu Val His Asn Ala Lys
                   325                 330                 335
   Thr Lys Pro Arg Glu Glu Gln Phe Gln Ser Thr Tyr Arg Val Val Ser
               340                 345                 350
   Val Leu Thr Val Leu His Gln Asp Trp Leu Asn Gly Lys Glu Tyr Lys
           355                 360                 365
   Cys Lys Val Ser Asn Lys Gly Leu Pro Ser Ser Ile Glu Lys Thr Ile
       370                 375                 380
   Ser Lys Ala Lys Gly Gln Pro Arg Glu Pro Gln Val Tyr Thr Leu Pro
   385                 390                 395                 400
   Pro Ser Gln Glu Glu Met Thr Lys Asn Gln Val Ser Leu Thr Cys Leu
                   405                 410                 415
   Val Lys Gly Phe Tyr Pro Ser Asp Ile Ala Val Glu Trp Glu Ser Asn
               420                 425                 430
   Gly Gln Pro Glu Asn Asn Tyr Lys Thr Thr Pro Pro Val Leu Asp Ser
           435                 440                 445
   Asp Gly Ser Phe Phe Leu Tyr Ser Arg Leu Thr Val Asp Lys Ser Arg
       450                 455                 460
   Trp Gln Glu Gly Asn Val Phe Ser Cys Ser Val Met His Glu Ala Leu
   465                 470                 475                 480
   His Asn His Tyr Thr Gln Lys Ser Leu Ser Leu Ser Leu Gly Lys Met
                   485                 490                 495
   Phe Trp Val Leu Val Val Val Gly Gly Val Leu Ala Cys Tyr Ser Leu
               500                 505                 510
   Leu Val Thr Val Ala Phe Ile Ile Phe Trp Val Lys Arg Gly Arg Lys
           515                 520                 525
   Lys Leu Leu Tyr Ile Phe Lys Gln Pro Phe Met Arg Pro Val Gln Thr
       530                 535                 540
   Thr Gln Glu Glu Asp Gly Cys Ser Cys Arg Phe Pro Glu Glu Glu Glu
   545                 550                 555                 560
   Gly Gly Cys Glu Leu Arg Val Lys Phe Ser Arg Ser Ala Asp Ala Pro
                   565                 570                 575
   Ala Tyr Gln Gln Gly Gln Asn Gln Leu Tyr Asn Glu Leu Asn Leu Gly
               580                 585                 590
   Arg Arg Glu Glu Tyr Asp Val Leu Asp Lys Arg Arg Gly Arg Asp Pro
           595                 600                 605
   Glu Met Gly Gly Lys Pro Arg Arg Lys Asn Pro Gln Glu Gly Leu Tyr
       610                 615                 620
   Asn Glu Leu Gln Lys Asp Lys Met Ala Glu Ala Tyr Ser Glu Ile Gly
   625                 630                 635                 640
   Met Lys Gly Glu Arg Arg Arg Gly Lys Gly His Asp Gly Leu Tyr Gln
                   645                 650                 655
   Gly Leu Ser Thr Ala Thr Lys Asp Thr Tyr Asp Ala Leu His Met Gln
                   660                 665                 670
   Ala Leu Pro Pro Arg Leu Glu Gly Gly Gly Glu Gly Arg Gly Ser Leu
               675                 680                 685
   Leu Thr Cys Gly Asp Val Glu Glu Asn Pro Gly Pro Arg Met Leu Leu
       690                 695                 700
   Leu Val Thr Ser Leu Leu Leu Cys Glu Leu Pro His Pro Ala Phe Leu
   705                 710                 715                 720
   Leu Ile Pro Arg Lys Val Cys Asn Gly Ile Gly Ile Gly Glu Phe Lys
                   725                 730                 735
   Asp Ser Leu Ser Ile Asn Ala Thr Asn Ile Lys His Phe Lys Asn Cys
                   740                 745                 750
   Thr Ser Ile Ser Gly Asp Leu His Ile Leu Pro Val Ala Phe Arg Gly
               755                 760                 765
   Asp Ser Phe Thr His Thr Pro Pro Leu Asp Pro Gln Glu Leu Asp Ile
       770                 775                 780
   Leu Lys Thr Val Lys Glu Ile Thr Gly Phe Leu Leu Ile Gln Ala Trp
   785                 790                 795                 800
   Pro Glu Asn Arg Thr Asp Leu His Ala Phe Glu Asn Leu Glu Ile Ile
                   805                 810                 815
   Arg Gly Arg Thr Lys Gln His Gly Gln Phe Ser Leu Ala Val Val Ser
                   820                 825                 830
   Leu Asn Ile Thr Ser Leu Gly Leu Arg Ser Leu Lys Glu Ile Ser Asp
               835                 840                 845
   Gly Asp Val Ile Ile Ser Gly Asn Lys Asn Leu Cys Tyr Ala Asn Thr
       850                 855                 860
   Ile Asn Trp Lys Lys Leu Phe Gly Thr Ser Gly Gln Lys Thr Lys Ile
   865                 870                 875                 880
   Ile Ser Asn Arg Gly Glu Asn Ser Cys Lys Ala Thr Gly Gln Val Cys
                   885                 890                 895
   His Ala Leu Cys Ser Pro Glu Gly Cys Trp Gly Pro Glu Pro Arg Asp
                   900                 905                 910
   Cys Val Ser Cys Arg Asn Val Ser Arg Gly Arg Glu Cys Val Asp Lys
               915                 920                 925
   Cys Asn Leu Leu Glu Gly Glu Pro Arg Glu Phe Val Glu Asn Ser Glu
       930                 935                 940
   Cys Ile Gln Cys His Pro Glu Cys Leu Pro Gln Ala Met Asn Ile Thr
   945                 950                 955                 960
   Cys Thr Gly Arg Gly Pro Asp Asn Cys Ile Gln Cys Ala His Tyr Ile
                   965                 970                 975
   Asp Gly Pro His Cys Val Lys Thr Cys Pro Ala Gly Val Met Gly Glu
                   980                 985                 990
   Asn Asn Thr Leu Val Trp Lys Tyr Ala Asp Ala Gly His Val Cys His
               995                 1000                1005
   Leu Cys His Pro Asn Cys Thr Tyr Gly Cys Thr Gly Pro Gly Leu Glu
       1010                1015                1020
   Gly Cys Pro Thr Asn Gly Pro Lys Ile Pro Ser Ile Ala Thr Gly Met
   1025                1030                1035                1040
   Val Gly Ala Leu Leu Leu Leu Leu Val Val Ala Leu Gly Ile Gly Leu
                   1045                1050                1055
   Phe Met


<210> 14
<211> 832
<212> PRT
<213> Artificial Sequence


<220>
<221> CHAIN
<222> 1..25
<223> signal peptide

<220>
<223> HLA-DRA*01:01 CLR short construct with Jun-Zipper

<220>
<221> CHAIN
<222> 26..109
<223> alpha 1 chain

<220>
<221> CHAIN
<222> 110..203
<223> alpha 2 chain

<220>
<221> CHAIN
<222> 204..216
<223> extrcellular part of transmembrane domain

<220>
<221> CHAIN
<222> 217..223
<223> linker

<220>
<221> CHAIN
<222> 224..257
<223> Jun-Zipper

<220>
<221> CHAIN
<222> 258..269
<223> short spacer domain

<220>
<221> CHAIN
<222> 270..297
<223> CD28 transmembrane domain

<220>
<221> CHAIN
<222> 298..339
<223> 4-1BB costimulatory domain

<220>
<221> CHAIN
<222> 340..451
<223> CD3 zeta signalling domain

<220>
<221> CHAIN
<222> 452..475
<223> T2A element

<220>
<221> CHAIN
<222> 476..497
<223> GM-CSF signal peptide

<220>
<221> CHAIN
<222> 498..832
<223> truncated EGFR selection domain

<400> 14
Met Ala Ile Ser Gly Val Pro Val Leu Gly Phe Phe Ile Ile Ala Val
1               5                   10                  15
Leu Met Ser Ala Gln Glu Ser Trp Ala Ile Lys Glu Glu His Val Ile
            20                  25                  30
Ile Gln Ala Glu Phe Tyr Leu Asn Pro Asp Gln Ser Gly Glu Phe Met
        35                  40                  45
Phe Asp Phe Asp Gly Asp Glu Ile Phe His Val Asp Met Ala Lys Lys
    50                  55                  60
Glu Thr Val Trp Arg Leu Glu Glu Phe Gly Arg Phe Ala Ser Phe Glu
65                  70                  75                  80
Ala Gln Gly Ala Leu Ala Asn Ile Ala Val Asp Lys Ala Asn Leu Glu
                85                  90                  95
Ile Met Thr Lys Arg Ser Asn Tyr Thr Pro Ile Thr Asn Val Pro Pro
            100                 105                 110
Glu Val Thr Val Leu Thr Asn Ser Pro Val Glu Leu Arg Glu Pro Asn
        115                 120                 125
Val Leu Ile Cys Phe Ile Asp Lys Phe Thr Pro Pro Val Val Asn Val
    130                 135                 140
Thr Trp Leu Arg Asn Gly Lys Pro Val Thr Thr Gly Val Ser Glu Thr
145                 150                 155                 160
Val Phe Leu Pro Arg Glu Asp His Leu Phe Arg Lys Phe His Tyr Leu
                165                 170                 175
Pro Phe Leu Pro Ser Thr Glu Asp Val Tyr Asp Cys Arg Val Glu His
            180                 185                 190
Trp Gly Leu Asp Glu Pro Leu Leu Lys His Trp Glu Phe Asp Ala Pro
        195                 200                 205
Ser Pro Leu Pro Glu Thr Thr Glu Ser Arg Gly Gly Gly Gly Gly Ala
    210                 215                 220
Arg Leu Glu Glu Lys Val Lys Thr Leu Lys Ala Gln Asn Ser Glu Leu
225                 230                 235                 240
Ala Ser Thr Ala Asn Met Leu Arg Glu Gln Val Ala Gln Leu Lys Gln
                245                 250                 255
Ser Glu Ser Lys Tyr Gly Pro Pro Cys Pro Pro Cys Pro Met Phe Trp
            260                 265                 270
Val Leu Val Val Val Gly Gly Val Leu Ala Cys Tyr Ser Leu Leu Val
        275                 280                 285
Thr Val Ala Phe Ile Ile Phe Trp Val Lys Arg Gly Arg Lys Lys Leu
    290                 295                 300
Leu Tyr Ile Phe Lys Gln Pro Phe Met Arg Pro Val Gln Thr Thr Gln
305                 310                 315                 320
Glu Glu Asp Gly Cys Ser Cys Arg Phe Pro Glu Glu Glu Glu Gly Gly
                325                 330                 335
Cys Glu Leu Arg Val Lys Phe Ser Arg Ser Ala Asp Ala Pro Ala Tyr
            340                 345                 350
Gln Gln Gly Gln Asn Gln Leu Tyr Asn Glu Leu Asn Leu Gly Arg Arg
        355                 360                 365
Glu Glu Tyr Asp Val Leu Asp Lys Arg Arg Gly Arg Asp Pro Glu Met
    370                 375                 380
Gly Gly Lys Pro Arg Arg Lys Asn Pro Gln Glu Gly Leu Tyr Asn Glu
385                 390                 395                 400
Leu Gln Lys Asp Lys Met Ala Glu Ala Tyr Ser Glu Ile Gly Met Lys
                405                 410                 415
Gly Glu Arg Arg Arg Gly Lys Gly His Asp Gly Leu Tyr Gln Gly Leu
            420                 425                 430
Ser Thr Ala Thr Lys Asp Thr Tyr Asp Ala Leu His Met Gln Ala Leu
        435                 440                 445
Pro Pro Arg Leu Glu Gly Gly Gly Glu Gly Arg Gly Ser Leu Leu Thr
    450                 455                 460
Cys Gly Asp Val Glu Glu Asn Pro Gly Pro Arg Met Leu Leu Leu Val
465                 470                 475                 480
Thr Ser Leu Leu Leu Cys Glu Leu Pro His Pro Ala Phe Leu Leu Ile

                485                 490                 495
Pro Arg Lys Val Cys Asn Gly Ile Gly Ile Gly Glu Phe Lys Asp Ser
            500                 505                 510
Leu Ser Ile Asn Ala Thr Asn Ile Lys His Phe Lys Asn Cys Thr Ser
        515                 520                 525
Ile Ser Gly Asp Leu His Ile Leu Pro Val Ala Phe Arg Gly Asp Ser
    530                 535                 540
Phe Thr His Thr Pro Pro Leu Asp Pro Gln Glu Leu Asp Ile Leu Lys
545                 550                 555                 560
Thr Val Lys Glu Ile Thr Gly Phe Leu Leu Ile Gln Ala Trp Pro Glu
                565                 570                 575
Asn Arg Thr Asp Leu His Ala Phe Glu Asn Leu Glu Ile Ile Arg Gly
            580                 585                 590
Arg Thr Lys Gln His Gly Gln Phe Ser Leu Ala Val Val Ser Leu Asn
        595                 600                 605
Ile Thr Ser Leu Gly Leu Arg Ser Leu Lys Glu Ile Ser Asp Gly Asp
    610                 615                 620
Val Ile Ile Ser Gly Asn Lys Asn Leu Cys Tyr Ala Asn Thr Ile Asn
625                 630                 635                 640
Trp Lys Lys Leu Phe Gly Thr Ser Gly Gln Lys Thr Lys Ile Ile Ser
                645                 650                 655
Asn Arg Gly Glu Asn Ser Cys Lys Ala Thr Gly Gln Val Cys His Ala
            660                 665                 670
Leu Cys Ser Pro Glu Gly Cys Trp Gly Pro Glu Pro Arg Asp Cys Val
        675                 680                 685
Ser Cys Arg Asn Val Ser Arg Gly Arg Glu Cys Val Asp Lys Cys Asn
    690                 695                 700
Leu Leu Glu Gly Glu Pro Arg Glu Phe Val Glu Asn Ser Glu Cys Ile
705                 710                 715                 720
Gln Cys His Pro Glu Cys Leu Pro Gln Ala Met Asn Ile Thr Cys Thr
                725                 730                 735
Gly Arg Gly Pro Asp Asn Cys Ile Gln Cys Ala His Tyr Ile Asp Gly
            740                 745                 750
Pro His Cys Val Lys Thr Cys Pro Ala Gly Val Met Gly Glu Asn Asn
        755                 760                 765
Thr Leu Val Trp Lys Tyr Ala Asp Ala Gly His Val Cys His Leu Cys
    770                 775                 780
His Pro Asn Cys Thr Tyr Gly Cys Thr Gly Pro Gly Leu Glu Gly Cys
785                 790                 795                 800
Pro Thr Asn Gly Pro Lys Ile Pro Ser Ile Ala Thr Gly Met Val Gly
                805                 810                 815
Ala Leu Leu Leu Leu Leu Val Val Ala Leu Gly Ile Gly Leu Phe Met
            820                 825                 830

<210> 15
<211> 267
<212> PRT
<213> Artificial Sequence

<220>
<221> CHAIN
<222> 1..29
<223> signal peptide

<220>
<223> HLA-DRB1*01:01 ligand with linker and Fos-Zipper

<220>
<221> CHAIN
<222> 30..123

<223> beta 1 domain

<220>
<221> CHAIN
<222> 124..217
<223> beta 2 domain

<220>
<221> CHAIN
<222> 218..227
<223> extracellular part of transmembrane domain

<220>
<221> CHAIN
<222> 228..234
<223> linker

<220>
<221> CHAIN
<222> 235..267
<223> Fos-Zipper

<400> 15
Met Val Cys Leu Lys Leu Pro Gly Gly Ser Cys Met Thr Ala Leu Thr
1               5                   10                  15
Val Thr Leu Met Val Leu Ser Ser Pro Leu Ala Leu Ala Gly Asp Thr
            20                  25                  30
Arg Pro Arg Phe Leu Trp Gln Leu Lys Phe Glu Cys His Phe Phe Asn
        35                  40                  45
Gly Thr Glu Arg Val Arg Leu Leu Glu Arg Cys Ile Tyr Asn Gln Glu
    50                  55                  60
Glu Ser Val Arg Phe Asp Ser Asp Val Gly Glu Tyr Arg Ala Val Thr
65                  70                  75                  80
Glu Leu Gly Arg Pro Asp Ala Glu Tyr Trp Asn Ser Gln Lys Asp Leu
                85                  90                  95
Leu Glu Gln Arg Arg Ala Ala Val Asp Thr Tyr Cys Arg His Asn Tyr
            100                 105                 110
Gly Val Gly Glu Ser Phe Thr Val Gln Arg Arg Val Glu Pro Lys Val
        115                 120                 125
Thr Val Tyr Pro Ser Lys Thr Gln Pro Leu Gln His His Asn Leu Leu
    130                 135                 140
Val Cys Ser Val Ser Gly Phe Tyr Pro Gly Ser Ile Glu Val Arg Trp
145                 150                 155                 160
Phe Arg Asn Gly Gln Glu Glu Lys Ala Gly Val Val Ser Thr Gly Leu
                165                 170                 175
Ile Gln Asn Gly Asp Trp Thr Phe Gln Thr Leu Val Met Leu Glu Thr
            180                 185                 190
Val Pro Arg Ser Gly Glu Val Tyr Thr Cys Gln Val Glu His Pro Ser
        195                 200                 205
Val Thr Ser Pro Leu Thr Val Glu Trp Arg Ala Arg Ser Glu Ser Ala
    210                 215                 220
Gln Ser Lys Ser Arg Gly Gly Gly Gly Gly Asp Thr Leu Gln Ala Glu
225                 230                 235                 240
Thr Asp Gln Leu Glu Asp Glu Lys Ser Ala Leu Gln Thr Glu Ile Ala
                245                 250                 255
Asn Leu Leu Lys Glu Lys Glu Lys Leu Glu Phe
            260                 265

<210> 16
<211> 1048
<212> PRT
<213> Artificial Sequence

<220>
<221> CHAIN
<222> 1..25
<223> signal peptide

<220>
<223> HLA-DRA*01:01 CLR long construct with Jun-Zipper

<220>
<221> CHAIN
<222> 26..109
<223> alpha 1 domain

<220>
<221> CHAIN
<222> 110..203
<223> alpha 2 domain

<220>
<221> CHAIN
<222> 204..216
<223> extracellular part of transmembrane domain

<220>
<221> CHAIN
<222> 217..223
<223> linker

<220>
<221> CHAIN
<222> 224..257
<223> Jun-Zipper

<220>
<221> CHAIN
<222> 258..485
<223> long spacer domain

<220>
<221> CHAIN
<222> 486..513
<223> CD28 transmembrane domain

<220>
<221> CHAIN
<222> 514..555
<223> 4-1BB costimulatory domain

<220>
<221> CHAIN
<222> 556..667
<223> CD3 zeta signalling domain

<220>
<221> CHAIN
<222> 668..691
<223> T2A element

<220>
<221> CHAIN
<222> 692..713
<223> GM-CSF signal peptied

<220>
<221> CHAIN
<222> 714..1048
<223> EGFR selection domain

<400> 16
Met Ala Ile Ser Gly Val Pro Val Leu Gly Phe Phe Ile Ile Ala Val
1               5                   10                  15
Leu Met Ser Ala Gln Glu Ser Trp Ala Ile Lys Glu Glu His Val Ile
            20                  25                  30
Ile Gln Ala Glu Phe Tyr Leu Asn Pro Asp Gln Ser Gly Glu Phe Met
        35                  40                  45
Phe Asp Phe Asp Gly Asp Glu Ile Phe His Val Asp Met Ala Lys Lys
    50                  55                  60
Glu Thr Val Trp Arg Leu Glu Glu Phe Gly Arg Phe Ala Ser Phe Glu
65                  70                  75                  80
Ala Gln Gly Ala Leu Ala Asn Ile Ala Val Asp Lys Ala Asn Leu Glu
                85                  90                  95
Ile Met Thr Lys Arg Ser Asn Tyr Thr Pro Ile Thr Asn Val Pro Pro
            100                 105                 110
Glu Val Thr Val Leu Thr Asn Ser Pro Val Glu Leu Arg Glu Pro Asn
        115                 120                 125
Val Leu Ile Cys Phe Ile Asp Lys Phe Thr Pro Pro Val Val Asn Val
    130                 135                 140
Thr Trp Leu Arg Asn Gly Lys Pro Val Thr Thr Gly Val Ser Glu Thr
145                 150                 155                 160
Val Phe Leu Pro Arg Glu Asp His Leu Phe Arg Lys Phe His Tyr Leu
                165                 170                 175
Pro Phe Leu Pro Ser Thr Glu Asp Val Tyr Asp Cys Arg Val Glu His
            180                 185                 190
Trp Gly Leu Asp Glu Pro Leu Leu Lys His Trp Glu Phe Asp Ala Pro
        195                 200                 205
Ser Pro Leu Pro Glu Thr Thr Glu Ser Arg Gly Gly Gly Gly Gly Ala
    210                 215                 220
Arg Leu Glu Glu Lys Val Lys Thr Leu Lys Ala Gln Asn Ser Glu Leu
225                 230                 235                 240
Ala Ser Thr Ala Asn Met Leu Arg Glu Gln Val Ala Gln Leu Lys Gln
                245                 250                 255
Ser Glu Ser Lys Tyr Gly Pro Pro Cys Pro Pro Cys Pro Ala Pro Pro
            260                 265                 270
Val Ala Gly Pro Ser Val Phe Leu Phe Pro Pro Lys Pro Lys Asp Thr
        275                 280                 285
Leu Met Ile Ser Arg Thr Pro Glu Val Thr Cys Val Val Val Asp Val
    290                 295                 300
Ser Gln Glu Asp Pro Glu Val Gln Phe Asn Trp Tyr Val Asp Gly Val
305                 310                 315                 320
Glu Val His Asn Ala Lys Thr Lys Pro Arg Glu Glu Gln Phe Gln Ser
                325                 330                 335
Thr Tyr Arg Val Val Ser Val Leu Thr Val Leu His Gln Asp Trp Leu
            340                 345                 350
Asn Gly Lys Glu Tyr Lys Cys Lys Val Ser Asn Lys Gly Leu Pro Ser
        355                 360                 365
Ser Ile Glu Lys Thr Ile Ser Lys Ala Lys Gly Gln Pro Arg Glu Pro
    370                 375                 380
Gln Val Tyr Thr Leu Pro Pro Ser Gln Glu Glu Met Thr Lys Asn Gln
385                 390                 395                 400
Val Ser Leu Thr Cys Leu Val Lys Gly Phe Tyr Pro Ser Asp Ile Ala
                405                 410                 415
Val Glu Trp Glu Ser Asn Gly Gln Pro Glu Asn Asn Tyr Lys Thr Thr
            420                 425                 430
Pro Pro Val Leu Asp Ser Asp Gly Ser Phe Phe Leu Tyr Ser Arg Leu
        435                 440                 445

Thr Val Asp Lys Ser Arg Trp Gln Glu Gly Asn Val Phe Ser Cys Ser
    450                 455                 460
Val Met His Glu Ala Leu His Asn His Tyr Thr Gln Lys Ser Leu Ser
465                 470                 475                 480
Leu Ser Leu Gly Lys Met Phe Trp Val Leu Val Val Val Gly Gly Val
                485                 490                 495
Leu Ala Cys Tyr Ser Leu Leu Val Thr Val Ala Phe Ile Ile Phe Trp
            500                 505                 510
Val Lys Arg Gly Arg Lys Lys Leu Leu Tyr Ile Phe Lys Gln Pro Phe
        515                 520                 525
Met Arg Pro Val Gln Thr Thr Gln Glu Glu Asp Gly Cys Ser Cys Arg
    530                 535                 540
Phe Pro Glu Glu Glu Glu Gly Gly Cys Glu Leu Arg Val Lys Phe Ser
545                 550                 555                 560
Arg Ser Ala Asp Ala Pro Ala Tyr Gln Gln Gly Gln Asn Gln Leu Tyr
                565                 570                 575
Asn Glu Leu Asn Leu Gly Arg Arg Glu Glu Tyr Asp Val Leu Asp Lys
            580                 585                 590
Arg Arg Gly Arg Asp Pro Glu Met Gly Gly Lys Pro Arg Arg Lys Asn
        595                 600                 605
Pro Gln Glu Gly Leu Tyr Asn Glu Leu Gln Lys Asp Lys Met Ala Glu
    610                 615                 620
Ala Tyr Ser Glu Ile Gly Met Lys Gly Glu Arg Arg Arg Gly Lys Gly
625                 630                 635                 640
His Asp Gly Leu Tyr Gln Gly Leu Ser Thr Ala Thr Lys Asp Thr Tyr
                645                 650                 655
Asp Ala Leu His Met Gln Ala Leu Pro Pro Arg Leu Glu Gly Gly Gly
            660                 665                 670
Glu Gly Arg Gly Ser Leu Leu Thr Cys Gly Asp Val Glu Glu Asn Pro
        675                 680                 685
Gly Pro Arg Met Leu Leu Leu Val Thr Ser Leu Leu Leu Cys Glu Leu
    690                 695                 700
Pro His Pro Ala Phe Leu Leu Ile Pro Arg Lys Val Cys Asn Gly Ile
705                 710                 715                 720
Gly Ile Gly Glu Phe Lys Asp Ser Leu Ser Ile Asn Ala Thr Asn Ile
                725                 730                 735
Lys His Phe Lys Asn Cys Thr Ser Ile Ser Gly Asp Leu His Ile Leu
            740                 745                 750
Pro Val Ala Phe Arg Gly Asp Ser Phe Thr His Thr Pro Pro Leu Asp
        755                 760                 765
Pro Gln Glu Leu Asp Ile Leu Lys Thr Val Lys Glu Ile Thr Gly Phe
    770                 775                 780
Leu Leu Ile Gln Ala Trp Pro Glu Asn Arg Thr Asp Leu His Ala Phe
785                 790                 795                 800
Glu Asn Leu Glu Ile Ile Arg Gly Arg Thr Lys Gln His Gly Gln Phe
                805                 810                 815
Ser Leu Ala Val Val Ser Leu Asn Ile Thr Ser Leu Gly Leu Arg Ser
            820                 825                 830
Leu Lys Glu Ile Ser Asp Gly Asp Val Ile Ile Ser Gly Asn Lys Asn
        835                 840                 845
Leu Cys Tyr Ala Asn Thr Ile Asn Trp Lys Lys Leu Phe Gly Thr Ser
    850                 855                 860
Gly Gln Lys Thr Lys Ile Ile Ser Asn Arg Gly Glu Asn Ser Cys Lys
865                 870                 875                 880
Ala Thr Gly Gln Val Cys His Ala Leu Cys Ser Pro Glu Gly Cys Trp
                885                 890                 895
Gly Pro Glu Pro Arg Asp Cys Val Ser Cys Arg Asn Val Ser Arg Gly
            900                 905                 910
Arg Glu Cys Val Asp Lys Cys Asn Leu Leu Glu Gly Glu Pro Arg Glu
        915                 920                 925
Phe Val Glu Asn Ser Glu Cys Ile Gln Cys His Pro Glu Cys Leu Pro
    930                 935                 940
Gln Ala Met Asn Ile Thr Cys Thr Gly Arg Gly Pro Asp Asn Cys Ile

945                 950                 955                 960
Gln Cys Ala His Tyr Ile Asp Gly Pro His Cys Val Lys Thr Cys Pro
                965                 970                 975
Ala Gly Val Met Gly Glu Asn Asn Thr Leu Val Trp Lys Tyr Ala Asp
            980                 985                 990
Ala Gly His Val Cys His Leu Cys His Pro Asn Cys Thr Tyr Gly Cys
        995                 1000                1005
Thr Gly Pro Gly Leu Glu Gly Cys Pro Thr Asn Gly Pro Lys Ile Pro
    1010                1015                1020
Ser Ile Ala Thr Gly Met Val Gly Ala Leu Leu Leu Leu Leu Val Val
1025                1030                1035                1040
Ala Leu Gly Ile Gly Leu Phe Met
                1045

<210> 17
<211> 267
<212> PRT
<213> Artificial Sequence

<220>
<221> CHAIN
<222> 1..29
<223> signal peptide

<220>
<223> HLA-DRB1*01:01 ligand with linker and Fos-Zipper

<220>
<221> CHAIN
<222> 30..123
<223> beta 1 domain

<220>
<221> CHAIN
<222> 124..217
<223> beta 2 domain

<220>
<221> CHAIN
<222> 218..227
<223> extracellular part of transmembrane domain

<220>
<221> CHAIN
<222> 228..234
<223> linker

<220>
<221> CHAIN
<222> 235..267
<223> Fos-Zipper

<400> 17
Met Val Cys Leu Lys Leu Pro Gly Gly Ser Cys Met Thr Ala Leu Thr
1               5                   10                  15
Val Thr Leu Met Val Leu Ser Ser Pro Leu Ala Leu Ala Gly Asp Thr
            20                  25                  30
Arg Pro Arg Phe Leu Trp Gln Leu Lys Phe Glu Cys His Phe Phe Asn
        35                  40                  45
Gly Thr Glu Arg Val Arg Leu Leu Glu Arg Cys Ile Tyr Asn Gln Glu
    50                  55                  60
Glu Ser Val Arg Phe Asp Ser Asp Val Gly Glu Tyr Arg Ala Val Thr

65                  70                  75                  80
Glu Leu Gly Arg Pro Asp Ala Glu Tyr Trp Asn Ser Gln Lys Asp Leu
                85                  90                  95
Leu Glu Gln Arg Arg Ala Ala Val Asp Thr Tyr Cys Arg His Asn Tyr
            100                 105                 110
Gly Val Gly Glu Ser Phe Thr Val Gln Arg Arg Val Glu Pro Lys Val
        115                 120                 125
Thr Val Tyr Pro Ser Lys Thr Gln Pro Leu Gln His His Asn Leu Leu
    130                 135                 140
Val Cys Ser Val Ser Gly Phe Tyr Pro Gly Ser Ile Glu Val Arg Trp
145                 150                 155                 160
Phe Arg Asn Gly Gln Glu Glu Lys Ala Gly Val Val Ser Thr Gly Leu
                165                 170                 175
Ile Gln Asn Gly Asp Trp Thr Phe Gln Thr Leu Val Met Leu Glu Thr
            180                 185                 190
Val Pro Arg Ser Gly Glu Val Tyr Thr Cys Gln Val Glu His Pro Ser
        195                 200                 205
Val Thr Ser Pro Leu Thr Val Glu Trp Arg Ala Arg Ser Glu Ser Ala
    210                 215                 220
Gln Ser Lys Ser Arg Gly Gly Gly Gly Gly Asp Thr Leu Gln Ala Glu
225                 230                 235                 240
Thr Asp Gln Leu Glu Asp Glu Lys Ser Ala Leu Gln Thr Glu Ile Ala
                245                 250                 255
Asn Leu Leu Lys Glu Lys Glu Lys Leu Glu Phe
            260                 265

<210> 18
<211> 842
<212> PRT
<213> Artificial Sequence



<220>
<221> CHAIN
<222> 1..29
<223> signal peptide

<220>
<223> HLA-DRB1*01:01 CLR short construct with Fos-Zipper

<220>
<221> CHAIN
<222> 30..123
<223> beta 1 domain

<220>
<221> CHAIN
<222> 124..217
<223> beta 2 domain

<220>
<221> CHAIN
<222> 218..227
<223> extracellular part of transmembrane domain

<220>
<221> CHAIN
<222> 228..234
<223> linker

<220>
<221> CHAIN
<222> 235..267

<223> Fos-Zipper

<220>
<221> CHAIN
<222> 268..279
<223> short spacer domain

<220>
<221> CHAIN
<222> 280..307
<223> CD28 transmembrane domain

<220>
<221> CHAIN
<222> 308..349
<223> 4-1BB costimulatory domain

<220>
<221> CHAIN
<222> 350..461
<223> CD3 zeta signalling domain

<220>
<221> CHAIN
<222> 462..485
<223> T2A element

<220>
<221> CHAIN
<222> 486..507
<223> GM-CSF signal peptide

<220>
<221> CHAIN
<222> 508..842
<223> truncated EGFR selection domain

<400> 18
Met Val Cys Leu Lys Leu Pro Gly Gly Ser Cys Met Thr Ala Leu Thr
1               5                   10                  15
Val Thr Leu Met Val Leu Ser Ser Pro Leu Ala Leu Ala Gly Asp Thr
            20                  25                  30
Arg Pro Arg Phe Leu Trp Gln Leu Lys Phe Glu Cys His Phe Phe Asn
        35                  40                  45
Gly Thr Glu Arg Val Arg Leu Leu Glu Arg Cys Ile Tyr Asn Gln Glu
    50                  55                  60
Glu Ser Val Arg Phe Asp Ser Asp Val Gly Glu Tyr Arg Ala Val Thr
65                  70                  75                  80
Glu Leu Gly Arg Pro Asp Ala Glu Tyr Trp Asn Ser Gln Lys Asp Leu
                85                  90                  95
Leu Glu Gln Arg Arg Ala Ala Val Asp Thr Tyr Cys Arg His Asn Tyr
            100                 105                 110
Gly Val Gly Glu Ser Phe Thr Val Gln Arg Arg Val Glu Pro Lys Val
        115                 120                 125
Thr Val Tyr Pro Ser Lys Thr Gln Pro Leu Gln His His Asn Leu Leu
    130                 135                 140
Val Cys Ser Val Ser Gly Phe Tyr Pro Gly Ser Ile Glu Val Arg Trp
145                 150                 155                 160
Phe Arg Asn Gly Gln Glu Glu Lys Ala Gly Val Val Ser Thr Gly Leu
                165                 170                 175
Ile Gln Asn Gly Asp Trp Thr Phe Gln Thr Leu Val Met Leu Glu Thr
            180                 185                 190
Val Pro Arg Ser Gly Glu Val Tyr Thr Cys Gln Val Glu His Pro Ser

195                 200                 205
Val Thr Ser Pro Leu Thr Val Glu Trp Arg Ala Arg Ser Glu Ser Ala
    210                 215                 220
Gln Ser Lys Ser Arg Gly Gly Gly Gly Gly Asp Thr Leu Gln Ala Glu
225                 230                 235                 240
Thr Asp Gln Leu Glu Asp Glu Lys Ser Ala Leu Gln Thr Glu Ile Ala
                245                 250                 255
Asn Leu Leu Lys Glu Lys Glu Lys Leu Glu Phe Glu Ser Lys Tyr Gly
            260                 265                 270
Pro Pro Cys Pro Pro Cys Pro Met Phe Trp Val Leu Val Val Val Gly
        275                 280                 285
Gly Val Leu Ala Cys Tyr Ser Leu Leu Val Thr Val Ala Phe Ile Ile
    290                 295                 300
Phe Trp Val Lys Arg Gly Arg Lys Lys Leu Leu Tyr Ile Phe Lys Gln
305                 310                 315                 320
Pro Phe Met Arg Pro Val Gln Thr Thr Gln Glu Glu Asp Gly Cys Ser
                325                 330                 335
Cys Arg Phe Pro Glu Glu Glu Glu Gly Gly Cys Glu Leu Arg Val Lys
            340                 345                 350
Phe Ser Arg Ser Ala Asp Ala Pro Ala Tyr Gln Gln Gly Gln Asn Gln
        355                 360                 365
Leu Tyr Asn Glu Leu Asn Leu Gly Arg Arg Glu Glu Tyr Asp Val Leu
    370                 375                 380
Asp Lys Arg Arg Gly Arg Asp Pro Glu Met Gly Gly Lys Pro Arg Arg
385                 390                 395                 400
Lys Asn Pro Gln Glu Gly Leu Tyr Asn Glu Leu Gln Lys Asp Lys Met
                405                 410                 415
Ala Glu Ala Tyr Ser Glu Ile Gly Met Lys Gly Glu Arg Arg Arg Gly
            420                 425                 430
Lys Gly His Asp Gly Leu Tyr Gln Gly Leu Ser Thr Ala Thr Lys Asp
        435                 440                 445
Thr Tyr Asp Ala Leu His Met Gln Ala Leu Pro Pro Arg Leu Glu Gly
    450                 455                 460
Gly Gly Glu Gly Arg Gly Ser Leu Leu Thr Cys Gly Asp Val Glu Glu
465                 470                 475                 480
Asn Pro Gly Pro Arg Met Leu Leu Leu Val Thr Ser Leu Leu Leu Cys
                485                 490                 495
Glu Leu Pro His Pro Ala Phe Leu Leu Ile Pro Arg Lys Val Cys Asn
            500                 505                 510
Gly Ile Gly Ile Gly Glu Phe Lys Asp Ser Leu Ser Ile Asn Ala Thr
        515                 520                 525
Asn Ile Lys His Phe Lys Asn Cys Thr Ser Ile Ser Gly Asp Leu His
    530                 535                 540
Ile Leu Pro Val Ala Phe Arg Gly Asp Ser Phe Thr His Thr Pro Pro
545                 550                 555                 560
Leu Asp Pro Gln Glu Leu Asp Ile Leu Lys Thr Val Lys Glu Ile Thr
                565                 570                 575
Gly Phe Leu Leu Ile Gln Ala Trp Pro Glu Asn Arg Thr Asp Leu His
            580                 585                 590
Ala Phe Glu Asn Leu Glu Ile Ile Arg Gly Arg Thr Lys Gln His Gly
        595                 600                 605
Gln Phe Ser Leu Ala Val Val Ser Leu Asn Ile Thr Ser Leu Gly Leu
    610                 615                 620
Arg Ser Leu Lys Glu Ile Ser Asp Gly Asp Val Ile Ile Ser Gly Asn
625                 630                 635                 640
Lys Asn Leu Cys Tyr Ala Asn Thr Ile Asn Trp Lys Lys Leu Phe Gly
                645                 650                 655
Thr Ser Gly Gln Lys Thr Lys Ile Ile Ser Asn Arg Gly Glu Asn Ser
            660                 665                 670
Cys Lys Ala Thr Gly Gln Val Cys His Ala Leu Cys Ser Pro Glu Gly
        675                 680                 685
Cys Trp Gly Pro Glu Pro Arg Asp Cys Val Ser Cys Arg Asn Val Ser
    690                 695                 700

Arg Gly Arg Glu Cys Val Asp Lys Cys Asn Leu Leu Glu Gly Glu Pro
705                 710                 715                 720
Arg Glu Phe Val Glu Asn Ser Glu Cys Ile Gln Cys His Pro Glu Cys
                725                 730                 735
Leu Pro Gln Ala Met Asn Ile Thr Cys Thr Gly Arg Gly Pro Asp Asn
            740                 745                 750
Cys Ile Gln Cys Ala His Tyr Ile Asp Gly Pro His Cys Val Lys Thr
        755                 760                 765
Cys Pro Ala Gly Val Met Gly Glu Asn Asn Thr Leu Val Trp Lys Tyr
    770                 775                 780
Ala Asp Ala Gly His Val Cys His Leu Cys His Pro Asn Cys Thr Tyr
785                 790                 795                 800
Gly Cys Thr Gly Pro Gly Leu Glu Gly Cys Pro Thr Asn Gly Pro Lys
                805                 810                 815
Ile Pro Ser Ile Ala Thr Gly Met Val Gly Ala Leu Leu Leu Leu Leu
            820                 825                 830
Val Val Ala Leu Gly Ile Gly Leu Phe Met
        835                 840

<210> 19
<211> 257
<212> PRT
<213> Artificial Sequence

<220>
<221> CHAIN
<222> 1..25
<223> signal peptide

<220>
<223> HLA-DRA*01:01 ligand with linker and Jun-Zipper

<220>
<221> CHAIN
<222> 26..109
<223> alpha 1 chain

<220>
<221> CHAIN
<222> 110..203
<223> alpha 2 chain

<220>
<221> CHAIN
<222> 204..216
<223> extracellular part of transmembrane domain

<220>
<221> CHAIN
<222> 217..223
<223> linker

<220>
<221> CHAIN
<222> 224..257
<223> Jun-Zipper

<400> 19
Met Ala Ile Ser Gly Val Pro Val Leu Gly Phe Phe Ile Ile Ala Val
1               5                   10                  15
Leu Met Ser Ala Gln Glu Ser Trp Ala Ile Lys Glu Glu His Val Ile
            20                  25                  30

Ile Gln Ala Glu Phe Tyr Leu Asn Pro Asp Gln Ser Gly Glu Phe Met
        35                  40                  45
Phe Asp Phe Asp Gly Asp Glu Ile Phe His Val Asp Met Ala Lys Lys
    50                  55                  60
Glu Thr Val Trp Arg Leu Glu Glu Phe Gly Arg Phe Ala Ser Phe Glu
65                  70                  75                  80
Ala Gln Gly Ala Leu Ala Asn Ile Ala Val Asp Lys Ala Asn Leu Glu
                85                  90                  95
Ile Met Thr Lys Arg Ser Asn Tyr Thr Pro Ile Thr Asn Val Pro Pro
            100                 105                 110
Glu Val Thr Val Leu Thr Asn Ser Pro Val Glu Leu Arg Glu Pro Asn
        115                 120                 125
Val Leu Ile Cys Phe Ile Asp Lys Phe Thr Pro Pro Val Val Asn Val
    130                 135                 140
Thr Trp Leu Arg Asn Gly Lys Pro Val Thr Thr Gly Val Ser Glu Thr
145                 150                 155                 160
Val Phe Leu Pro Arg Glu Asp His Leu Phe Arg Lys Phe His Tyr Leu
                165                 170                 175
Pro Phe Leu Pro Ser Thr Glu Asp Val Tyr Asp Cys Arg Val Glu His
            180                 185                 190
Trp Gly Leu Asp Glu Pro Leu Leu Lys His Trp Glu Phe Asp Ala Pro
        195                 200                 205
Ser Pro Leu Pro Glu Thr Thr Glu Ser Arg Gly Gly Gly Gly Gly Ala
    210                 215                 220
Arg Leu Glu Glu Lys Val Lys Thr Leu Lys Ala Gln Asn Ser Glu Leu
225                 230                 235                 240
Ala Ser Thr Ala Asn Met Leu Arg Glu Gln Val Ala Gln Leu Lys Gln
                245                 250                 255
Ser


<210> 20
<211> 1058
<212> PRT
<213> Artificial Sequence



<220>
<221> CHAIN
<222> 1..29
<223> signal peptide

<220>
<223> HLA-DRB1*01:01 CLR long construct with Fos-Zipper

<220>
<221> CHAIN
<222> 30..123
<223> beta 1 domain

<220>
<221> CHAIN
<222> 124..217
<223> beta 2 domain

<220>
<221> CHAIN
<222> 218..227
<223> extracellular part of transmembrane domain

<220>
<221> CHAIN
<222> 228..234

<223> linker

<220>
<221> CHAIN
<222> 235..267
<223> Fos-Zipper

<220>
<221> CHAIN
<222> 268..495
<223> long spacer domain

<220>
<221> CHAIN
<222> 496..523
<223> CD28 transmembrane domain

<220>
<221> CHAIN
<222> 524..565
<223> 4-1BB costimulatory domain

<220>
<221> CHAIN
<222> 566..677
<223> CD3 zeta signalling domain

<220>
<221> CHAIN
<222> 678..701
<223> T2A element

<220>
<221> CHAIN
<222> 702..723
<223> GM-CSF signal peptide

<220>
<221> CHAIN
<222> 724..1058
<223> EGFR selection domain

<400> 20
Met Val Cys Leu Lys Leu Pro Gly Gly Ser Cys Met Thr Ala Leu Thr
1               5                   10                  15
Val Thr Leu Met Val Leu Ser Ser Pro Leu Ala Leu Ala Gly Asp Thr
            20                  25                  30
Arg Pro Arg Phe Leu Trp Gln Leu Lys Phe Glu Cys His Phe Phe Asn
        35                  40                  45
Gly Thr Glu Arg Val Arg Leu Leu Glu Arg Cys Ile Tyr Asn Gln Glu
    50                  55                  60
Glu Ser Val Arg Phe Asp Ser Asp Val Gly Glu Tyr Arg Ala Val Thr
65                  70                  75                  80
Glu Leu Gly Arg Pro Asp Ala Glu Tyr Trp Asn Ser Gln Lys Asp Leu
                85                  90                  95
Leu Glu Gln Arg Arg Ala Ala Val Asp Thr Tyr Cys Arg His Asn Tyr
            100                 105                 110
Gly Val Gly Glu Ser Phe Thr Val Gln Arg Arg Val Glu Pro Lys Val
        115                 120                 125
Thr Val Tyr Pro Ser Lys Thr Gln Pro Leu Gln His His Asn Leu Leu
    130                 135                 140
Val Cys Ser Val Ser Gly Phe Tyr Pro Gly Ser Ile Glu Val Arg Trp
145                 150                 155                 160

Phe Arg Asn Gly Gln Glu Glu Lys Ala Gly Val Val Ser Thr Gly Leu
                165                 170                 175
Ile Gln Asn Gly Asp Trp Thr Phe Gln Thr Leu Val Met Leu Glu Thr
            180                 185                 190
Val Pro Arg Ser Gly Glu Val Tyr Thr Cys Gln Val Glu His Pro Ser
        195                 200                 205
Val Thr Ser Pro Leu Thr Val Glu Trp Arg Ala Arg Ser Glu Ser Ala
    210                 215                 220
Gln Ser Lys Ser Arg Gly Gly Gly Gly Gly Asp Thr Leu Gln Ala Glu
225                 230                 235                 240
Thr Asp Gln Leu Glu Asp Glu Lys Ser Ala Leu Gln Thr Glu Ile Ala
                245                 250                 255
Asn Leu Leu Lys Glu Lys Glu Lys Leu Glu Phe Glu Ser Lys Tyr Gly
            260                 265                 270
Pro Pro Cys Pro Pro Cys Pro Ala Pro Pro Val Ala Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Gln Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys Met
                485                 490                 495
Phe Trp Val Leu Val Val Val Gly Gly Val Leu Ala Cys Tyr Ser Leu
            500                 505                 510
Leu Val Thr Val Ala Phe Ile Ile Phe Trp Val Lys Arg Gly Arg Lys
        515                 520                 525
Lys Leu Leu Tyr Ile Phe Lys Gln Pro Phe Met Arg Pro Val Gln Thr
    530                 535                 540
Thr Gln Glu Glu Asp Gly Cys Ser Cys Arg Phe Pro Glu Glu Glu Glu
545                 550                 555                 560
Gly Gly Cys Glu Leu Arg Val Lys Phe Ser Arg Ser Ala Asp Ala Pro
                565                 570                 575
Ala Tyr Gln Gln Gly Gln Asn Gln Leu Tyr Asn Glu Leu Asn Leu Gly
            580                 585                 590
Arg Arg Glu Glu Tyr Asp Val Leu Asp Lys Arg Arg Gly Arg Asp Pro
        595                 600                 605
Glu Met Gly Gly Lys Pro Arg Arg Lys Asn Pro Gln Glu Gly Leu Tyr
    610                 615                 620
Asn Glu Leu Gln Lys Asp Lys Met Ala Glu Ala Tyr Ser Glu Ile Gly
625                 630                 635                 640
Met Lys Gly Glu Arg Arg Arg Gly Lys Gly His Asp Gly Leu Tyr Gln
                645                 650                 655
Gly Leu Ser Thr Ala Thr Lys Asp Thr Tyr Asp Ala Leu His Met Gln

                660                 665                 670
Ala Leu Pro Pro Arg Leu Glu Gly Gly Gly Glu Gly Arg Gly Ser Leu
        675                 680                 685
Leu Thr Cys Gly Asp Val Glu Glu Asn Pro Gly Pro Arg Met Leu Leu
    690                 695                 700
Leu Val Thr Ser Leu Leu Leu Cys Glu Leu Pro His Pro Ala Phe Leu
705                 710                 715                 720
Leu Ile Pro Arg Lys Val Cys Asn Gly Ile Gly Ile Gly Glu Phe Lys
                725                 730                 735
Asp Ser Leu Ser Ile Asn Ala Thr Asn Ile Lys His Phe Lys Asn Cys
            740                 745                 750
Thr Ser Ile Ser Gly Asp Leu His Ile Leu Pro Val Ala Phe Arg Gly
        755                 760                 765
Asp Ser Phe Thr His Thr Pro Pro Leu Asp Pro Gln Glu Leu Asp Ile
    770                 775                 780
Leu Lys Thr Val Lys Glu Ile Thr Gly Phe Leu Leu Ile Gln Ala Trp
785                 790                 795                 800
Pro Glu Asn Arg Thr Asp Leu His Ala Phe Glu Asn Leu Glu Ile Ile
                805                 810                 815
Arg Gly Arg Thr Lys Gln His Gly Gln Phe Ser Leu Ala Val Val Ser
            820                 825                 830
Leu Asn Ile Thr Ser Leu Gly Leu Arg Ser Leu Lys Glu Ile Ser Asp
        835                 840                 845
Gly Asp Val Ile Ile Ser Gly Asn Lys Asn Leu Cys Tyr Ala Asn Thr
    850                 855                 860
Ile Asn Trp Lys Lys Leu Phe Gly Thr Ser Gly Gln Lys Thr Lys Ile
865                 870                 875                 880
Ile Ser Asn Arg Gly Glu Asn Ser Cys Lys Ala Thr Gly Gln Val Cys
                885                 890                 895
His Ala Leu Cys Ser Pro Glu Gly Cys Trp Gly Pro Glu Pro Arg Asp
            900                 905                 910
Cys Val Ser Cys Arg Asn Val Ser Arg Gly Arg Glu Cys Val Asp Lys
        915                 920                 925
Cys Asn Leu Leu Glu Gly Glu Pro Arg Glu Phe Val Glu Asn Ser Glu
    930                 935                 940
Cys Ile Gln Cys His Pro Glu Cys Leu Pro Gln Ala Met Asn Ile Thr
945                 950                 955                 960
Cys Thr Gly Arg Gly Pro Asp Asn Cys Ile Gln Cys Ala His Tyr Ile
                965                 970                 975
Asp Gly Pro His Cys Val Lys Thr Cys Pro Ala Gly Val Met Gly Glu
            980                 985                 990
Asn Asn Thr Leu Val Trp Lys Tyr Ala Asp Ala Gly His Val Cys His
        995                 1000                1005
Leu Cys His Pro Asn Cys Thr Tyr Gly Cys Thr Gly Pro Gly Leu Glu
    1010                1015                1020
Gly Cys Pro Thr Asn Gly Pro Lys Ile Pro Ser Ile Ala Thr Gly Met
1025                1030                1035                1040
Val Gly Ala Leu Leu Leu Leu Leu Val Val Ala Leu Gly Ile Gly Leu
                1045                1050                1055
Phe Met


<210> 21
<211> 257
<212> PRT
<213> Artificial Sequence


<220>
<221> CHAIN
<222> 1..25
<223> signal peptide

<220>
<223> HLA-DRA*01:01 Ligand with linker and Jun-Zipper

<220>
<221> CHAIN
<222> 26..109
<223> alpha 1 domain

<220>
<221> CHAIN
<222> 110..203
<223> alpha 2 domain

<220>
<221> CHAIN
<222> 204..216
<223> extracellular part of transmembrane domain

<220>
<221> CHAIN
<222> 217..223
<223> linker

<220>
<221> CHAIN
<222> 224..257
<223> Jun-Zipper

<400> 21
Met Ala Ile Ser Gly Val Pro Val Leu Gly Phe Phe Ile Ile Ala Val
1               5                   10                  15
Leu Met Ser Ala Gln Glu Ser Trp Ala Ile Lys Glu Glu His Val Ile
            20                  25                  30
Ile Gln Ala Glu Phe Tyr Leu Asn Pro Asp Gln Ser Gly Glu Phe Met
        35                  40                  45
Phe Asp Phe Asp Gly Asp Glu Ile Phe His Val Asp Met Ala Lys Lys
    50                  55                  60
Glu Thr Val Trp Arg Leu Glu Glu Phe Gly Arg Phe Ala Ser Phe Glu
65                  70                  75                  80
Ala Gln Gly Ala Leu Ala Asn Ile Ala Val Asp Lys Ala Asn Leu Glu
                85                  90                  95
Ile Met Thr Lys Arg Ser Asn Tyr Thr Pro Ile Thr Asn Val Pro Pro
            100                 105                 110
Glu Val Thr Val Leu Thr Asn Ser Pro Val Glu Leu Arg Glu Pro Asn
        115                 120                 125
Val Leu Ile Cys Phe Ile Asp Lys Phe Thr Pro Pro Val Val Asn Val
    130                 135                 140
Thr Trp Leu Arg Asn Gly Lys Pro Val Thr Thr Gly Val Ser Glu Thr
145                 150                 155                 160
Val Phe Leu Pro Arg Glu Asp His Leu Phe Arg Lys Phe His Tyr Leu
                165                 170                 175
Pro Phe Leu Pro Ser Thr Glu Asp Val Tyr Asp Cys Arg Val Glu His
            180                 185                 190
Trp Gly Leu Asp Glu Pro Leu Leu Lys His Trp Glu Phe Asp Ala Pro
        195                 200                 205
Ser Pro Leu Pro Glu Thr Thr Glu Ser Arg Gly Gly Gly Gly Gly Ala
    210                 215                 220
Arg Leu Glu Glu Lys Val Lys Thr Leu Lys Ala Gln Asn Ser Glu Leu
225                 230                 235                 240
Ala Ser Thr Ala Asn Met Leu Arg Glu Gln Val Ala Gln Leu Lys Gln
                245                 250                 255
Ser

## Description

The present invention relates to an artificial signalling molecule, which is a fusion protein, for expression in an immune cell, specifically for use in medical treatment. The artificial signalling molecule and an immune cell expressing the artificial signalling molecule are suitable for use in the treatment of undesired immune responses, e.g. for suppression of undesired immune activity, specifically for use in the treatment of immune rejections against transplants, e.g. treatment of HvG disease, for use in the treatment of autoimmune diseases, or for use in the treatment of allergies.

The artificial signalling molecule is designed to direct the immune cell expressing it against immune cells, e.g. T-cells, B-cells or NK cells, which cause the undesired immune response.

### State of the art

For suppression of undesired immune activities, it is generally known to suppress the general activity of the immune system.

WO 2018/001874 describes CAR molecules for expression in Treg cells for use in the treatment of HvG disease in a transplant recipient, wherein the CAR molecules generate a suppressive activity in the vicinity of the transplant.

### Object of the invention

It is an object of the invention to provide an alternative for use in the treatment of undesired immune activity, especially for treatment of undesired immune responses which are directed against specific cognate antigens, e.g. for use in the treatment of HvG disease, in the treatment of autoimmune disease, or for treatment of an allergy.

### Description of the invention

The invention achieves the object by the features of the claims, especially by providing a signalling molecule and an immune cell expressing the signalling molecule for use in the treatment of an undesired immune activity, which signalling molecule is a fusion protein which comprises or consists of a ligand domain, a spacer, a transmembrane domain, and at least one intracellular signalling domain, wherein the ligand domain comprises at least one epitope or all of the epitopes of the cognate antigen, or the cognate antigen, which cognate antigen is the target of the undesired immune activity. Preferably, especially in heterodimeric ligand domains with the two ligand domains, which are e.g. similar in size, the signalling molecule contains a dimerization domain arranged in its extracellular portion, e.g. arranged between the ligand domain and the transmembrane domain. Preferably, the domains of the signalling molecule, which comprise or consist of the ligand domain, a spacer, a transmembrane domain, and at least one intracellular signalling domain, optionally a dimerization domain between the ligand domain and the transmembrane domain, preferably the extracellular part of a HLA transmembrane domain at the C-terminus of the ligand domain, preferably a linker, e.g. of 8 to 12 amino acids, between the extracellular part of the HLA transmembrane domain and the dimerization domain or the spacer, are linked to one another from N-terminus to C-terminus, more preferably directly linked to one another form N-terminus to C-terminus. The immune cell expressing the signalling molecule contains a nucleic acid sequence encoding the signalling molecule, which nucleic acid sequence preferably is integrated into the cellular genome, e.g. by transduction using a viral vector, or by transfection of an encoding nucleic acid sequence.

The ligand domain accordingly comprises or consists of at least one, preferably all, of the epitopes, of the cognate antigen, e.g. the ligand domain can comprise or consist of the cognate antigen for a T-cell receptor (TCR) and/or for a B-cell receptor (BCR). The undesired immune activity to be treated is directed against the cognate antigen. As the signalling molecule of the invention for its specificity contains at least one epitope of the cognate antigen, against which the undesired immune response is directed, binding of a T-cell or B-cell or a NK cell to the ligand domain of the signalling molecule which is expressed by a CD4+ T-cell or a CD8+ T-cell, results in activating the T-cell effector functions specifically against the T-cell or B-cell or NK cell that is bound to the ligand domain of the signalling molecule. As a result of the binding of a CD4+ T-cell or a CD8+ T-cell via its TCR or of a B-cell via its BCR or of a NK cell via its receptor to the signalling molecule when expressed in a CD4+ T-cell or CD8+ T-cell, the bound T-cell or B-cell or NK cell is inactivated or killed, depending on the functional properties of the signalling T-cell. As the binding of a TCR of a T-cell or of a BCR of a B-cell or a KIR of a NK cell is mediated by the ligand domain of the signalling molecule, the inactivation or elimination of the bound T-cell or bound B-cell or bound NK cell is specific for the cognate antigen targeted by the specific receptor of the T-cell or B-cell or NK cell. Accordingly, herein the signalling molecule is also referred to as chimeric ligand receptor (CLR).

As the ligand domain provides a cognate antigen for a TCR or BCR, the ligand domain is no antibody, e.g. no scFv and no other antibody format.

Accordingly, for use in the treatment of HvG disease, the ligand domain, also referred to as the cognate antigen, can be the antigen against which an immune response against a transplant is directed, e.g. the ligand domain, respectively cognate antigen, is a heterologous, e.g. unmatched, HLA class I or class II molecule of the transplant.

Optionally, for use in the treatment of HvG disease, an immune cell expressing the signalling molecule can be for use in the treatment of a patient prior or following to transplantation, especially when the transplant is HLA-mismatched. Further, the immune cell expressing the signalling molecule can be for use in the treatment of a patient who after receiving a transplant has or has not generated memory B-cells directed against mismatched HLA molecules of the transplant.

For use in the treatment of the undesired immune response against a self-antigen, i.e. for use in the treatment of an autoimmune disease, the ligand domain, respectively cognate antigen, can be the self-antigen against which the autoimmune response is directed.
For use in the treatment of an allergy, the ligand domain, respectively cognate antigen, can be the cognate antigen of the allergy, e.g. a peanut antigen such as Ara h 1 (UniProtKB/Swiss-Prot: P43237.1).

The cognate antigen can e.g. be determined as the target of an antibody, of a B-cell receptor (BCR), of a T-cell receptor (TCR) or of a NK cell receptor (e.g. KIR), which occurs during or is characteristic of the undesired immune response. Preferably, the cognate antigen is predetermined directly or indirectly from a biopsy, e.g. a blood sample, obtained from the patient. For indirect pre-determination of the cognate antigen, the binding of antibody or T-cells of a biopsy can be analysed. For use in the treatment of HvG disease, the cognate antigen can be determined as the non-matched HLA molecule. The TCR of a T-cell, and respectively the BCR of a B-cell, generally are natural components of the patient who has the undesired immune response.

The at least one intracellular signalling domain is a domain which upon reaction of the antigen domain in T-cells activates T-cell effector functions. The intracellular signalling domain can e.g. be a hCD3ζ domain, preferably a combination of a h4-1BB domain and a hCD3ζ (zeta) domain, or a combination of an intracellular hCD28 signalling domain and a hCD3ζ domain.

The transmembrane domain can e.g. be a transmembrane domain of human CD28 (hCD28) or of CD4 (hCD4).

The immune cell expressing the signalling molecule is a T-cell, which preferably is immunologically compatible with the recipient, e.g. autologous to the recipient. In the alternative, the immune cell expressing the signalling molecule is not immunologically compatible with the recipient, e.g. is not HLA-matched with the recipient, so that following administration of the non-compatible immune cell expressing the signalling molecule this may eventually be eliminated by the immune system of the host when it has not been genetically modified in such a way that allorecognition of this immune cell is prevented by e.g. HLA knock down or knock out. Accordingly, for a T-cell which is not immunologically compatible with the recipient, e.g. is not HLA-matched with the recipient, the T-cell may be genetically modified not to express HLA molecules, e.g. genetically modified for knock down or knock out of expression of HLA molecules.

The immune cell expressing the signalling molecule is e.g. a primary CD4+ T-cell or a primary CD8+ T-cell, NK cell and/or a progenitor cell of one of these or a cytotoxic cell line, e.g. NK-92. Herein, the immune cell expressing the signalling molecule is also referred to as a T-cell, which represents a primary CD4+ T-cell or a primary CD8+ T-cell, an NK cell, and a progenitor cell of one of these, or a cytotoxic cell line.

The use of the signalling molecule, and of T-cells expressing the signalling molecule, in the treatment is that the B-cells and the T-cells and the NK cells of the recipient which specifically bind to the antigen domain of the signalling molecule are eliminated, while leaving unaffected other endogenous immune cells of the recipient. Accordingly, the use of the signalling molecule, and of T-cells expressing the signalling molecule, in medical treatment results in the selective inactivation in case of regulatory T cells or elimination of immune cells in case of cytotoxic T cells depending on their cognate receptor matching the ligand domain of the signalling molecule, without a general suppression of the immune system.

A dimerization domain, e.g. a zipper domain, is preferred for signalling molecules that as their ligand domain comprise or consist of two chains, e.g. an alpha chain and a beta chain, of a HLA class II molecule. For example, a first signalling molecule may comprise or consist of, preferably from N-terminus to C-terminus, an antigen domain, a linker, a dimerization domain (also termed zipper), a spacer, a transmembrane domain, and at least one intracellular signalling domain, wherein the dimerization domain is dimerized with the dimerization domain of a second signalling molecule, and wherein the antigen domains of the first and second signalling molecules may be different, e.g. alpha chain and beta chain of a HLA class II molecule. Therein, the second signalling molecule may comprise or consist of a chain of a HLA class II molecule as its ligand domain, a linker, a dimerization domain, a spacer, a transmembrane domain, and at least one intracellular signalling domain. In another embodiment, the second signalling molecule may comprise or consist of a chain of a HLA class II molecule as its ligand domain, a linker and a dimerization domain and is bound by the dimerized dimerization domains to the transmembrane domain and the at least one intracellular signalling domain of the first signalling molecule.

Generally, for a HLA class II molecule as the ligand domain, the ligand domain can consist of the alpha 1 domain and the beta 1 domain, and optionally the alpha 2 and the beta 2 domains, of the HLA class II heavy chains.

It was found that the arrangement of the extracellular part of the HLA transmembrane domain (in the sequences examples are referred to as extracellular part of transmembrane domain) at the C-terminal end of the alpha 2 domain or of the beta 2 domain results in a more stable molecule. The extracellular part of the HLA transmembrane domain is preferably encoded by exon 4 of a HLA class II and exon 5 of HLA class I encoding gene.

Optionally, for signalling molecules having the heavy chain (alpha chain) of HLA class I as the ligand domain, a β2-microglobulin may be bound by a dimerization domain, wherein e.g. the β2-microglobulin has a dimerization domain at its N-terminus or at its C-terminus. The signalling molecule may comprise or consist of, preferably from N-terminus to C-terminus, a ligand domain which is the heavy chain of a HLA class I molecule, optionally the extracellular part of the HLA transmembrane domain, a linker, a dimerization domain, a spacer, a transmembrane domain, and at least one intracellular signalling domain, wherein the dimerization domain is dimerized with the dimerization domain of a second molecule, which comprises or consists of β2-microglobulin having a dimerization domain at its N-terminus or at its C-terminus.

Herein, the spacer is also referred to as a hinge domain.

Generally, the ligand domain can be a MHC I molecule comprising an alpha chain and a β2-microglobulin according to European patent application No. 19166923.3, filed on 2. April 2019.

Generally, for HLA class I as ligand domain, the ligand domain can consist of the alpha 1 domain and the alpha 2 domain, and optionally the alpha 3 domain, of a HLA class I molecule.

Optionally, the T-cell expressing the CLR can encode two or more different CLR molecules, each having a different ligand domain, e.g. from different HLA class I or different HLA class II molecules or different HLA class I and HLA class II molecules. For HLA class I as the ligand domains the CLR is consisting of at least one, two or three HLA class I heavy chain domains, e.g. of the alpha 1 domain, the alpha 2 domain, and the alpha 3 domain. The light chain, i.e. the beta-2-microglobulin, can be the naturally expressed light chain or a recombinant beta-2-microgloblin, which could also be covalently bound to one of the heavy chain domains. For HLA class II as the ligand domains, the ligand domain can consist of at least one or two alpha chain domains, e.g. of the alpha 1 domain and alpha 2 domain and of at least one or two beta chain domains, e.g. of the beta 1 domain and the beta 2 domain.

The HLA class I or HLA class II antigens are known, e.g. from https://www.ebi.ac.uk/ipd/imgt/hla/download.html, release 0.3.35 or later, which can be downloaded from ftp://ftp.ebi.ac.uk/pub/databases/ipd/imgt/hla/.

Optionally, the signal molecule which as the ligand domain comprises an alpha chain or heavy chain of a HLA class I molecule, this ligand domain can contain at least one mutation in amino acid position No. 74, 223, 224, 225, 226, 227, 229, and 245, wherein the numbering refers to the mature protein, i.e. without the signal peptide, of the alpha chain or heavy chain of a HLA class I molecule, e.g. at least one mutation of 74L, 223A, 224F, 225D, 226A, 227K, 229A, and 245V, in order to reduce or abolish binding of the CD8 of CD8+ T-cells for reducing or abolishing a cytotoxic activity of CD8+ T-cells against T-cells expressing a CLR containing portion of a HLA class I molecule as its ligand domain.

Optionally, the signal molecule which as the ligand domain comprises an alpha and/or beta chain or heavy chains of a HLA class II molecule, this ligand domain can contain at least one mutation in amino acid position Nos. 88, 90 and 176, wherein the numbering refers to the mature protein, i.e. without the signal peptide, of the alpha chain of a HLA class II molecule, resulting in a weakening or abolishing of the interaction of DR od DQ or DP alpha chain with CD4+ T-cells, and/or a mutation in at least one amino acid position of Nos. 46, 54, 55, 56, 104, 114, 116, 134, 135, 136, 137, 138, 139, 141, 142, 143, 144, 145, 148, 158, 160, and 162 , wherein the numbering refers to the mature protein, i.e. without the signal peptide, of the beta chain of a HLA class II molecule, in order to weaken or abolish the interaction of DR or DQ or DP beta chain with CD4+ T-cells.

Optionally, the immune cell expressing the at least one signal molecule can further be genetically manipulated to additionally express at least one of the complement inhibitors hDAF (CD55), CD46, or CD59, or a combination of at least two of these, especially for use in the treatment of HvG disease or for use in the treatment of adverse immune reactions of a HLA-mismatched transplant.

The invention is now described by way of examples and with reference to figures, which show in
- Fig. 1 a schematic drawing of an embodiment of a signalling molecule of the invention,
- Fig. 2 a schematic drawing of a further embodiment of a signalling molecule of the invention,
- Fig. 3 a schematic drawing of a further embodiment of a signalling molecule of the invention,
- Fig. 4 a schematic drawing of a further embodiment of a signalling molecule of the invention,
- Fig. 5 in A), B), C), D) and E) schematic drawings of embodiments of the arrangement of domains of the signalling molecule,
- Fig. 6 the FACS result of detecting the CLR when expressed in immune cells by antibody directed against the antigen domain of the CLR,
- Fig. 7A) the FACS results of detecting the CLR when expressed in control cells (K562) and 7B) when expressed in immune cells (T-cells) by antibody directed against the antigen domain of the CLR,
- Fig. 8 LDH release as a measure for cytotoxicity of T-cells expressing the CLR, and
- Fig. 9A), B) and C) results of analysis of activation marker expression in T-cells expressing the CLR.

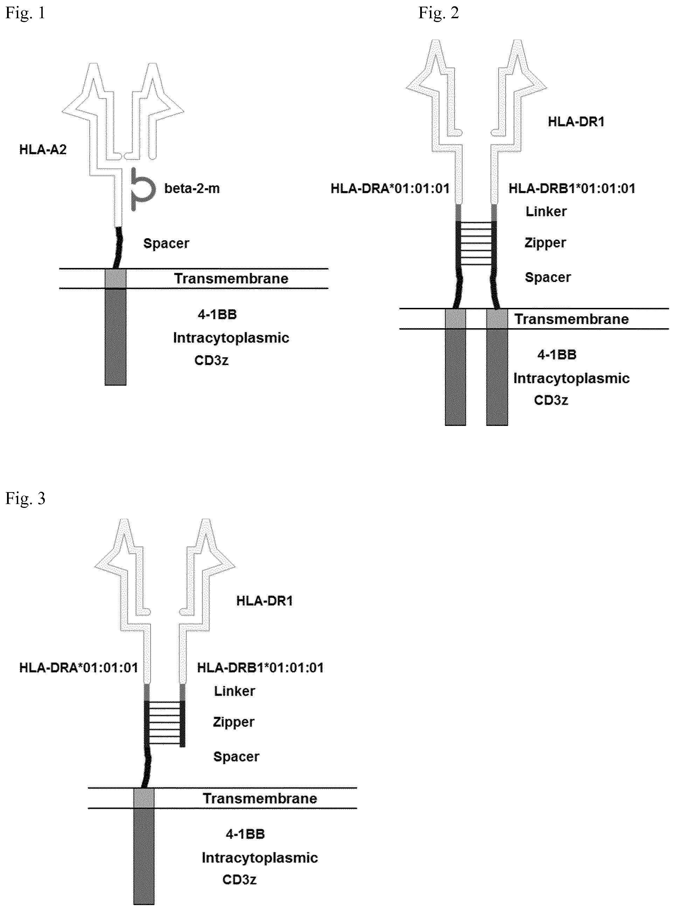

Fig. 1 shows a schematic drawing of a signalling molecule, CLR, of the invention, which is arranged in the membrane of a cellular membrane and consists of an extracellular ligand domain, e.g. a HLA class I molecule, in this example represented by the non-covalently bound light chain beta-2-micrglobulin, a HLA-A^{∗}02:01 heavy chain (HLA-A2) consisting of its alpha 1, alpha 2 and alpha 3 domains, and a spacer, a transmembrane domain spanning the cellular membrane, and an intracellularly arranged signalling domain of a combination of the intracellular 4-1BB domain and the CD3ζ (CD3z) domain. This exemplary ligand domain represents the cognate antigen of a HvG disease.

Fig. 2 shows a schematic drawing of a signalling molecule, CLR, of the invention, which comprises two heavy chains and which in a preferred embodiment having an extracellular dimerization domain represented by the two partners of a molecular zipper separated by a linker from each heavy chain and wherein both heavy chains via transmembrane domains are anchored in the membrane of a cellular membrane. This signalling molecule consists of two proteins that are dimerized by their dimerization domains, each containing an extracellular ligand domain, e.g. a HLA class II molecule, in this example represented by a HLA-DR1 antigen consisting of its alpha 1 and alpha 2 domains of the HLA-DR1 alpha chain HLA-DRA^{∗}01:01 and the beta 1 and beta 2 domains of the HLA-DR1 beta chain HLA-DRB1^{∗}01:01, with each heavy chain linked to a spacer, a transmembrane domain spanning the cellular membrane, and an intracellularly arranged signalling domain of a combination of the intracellular 4-1BB domain and the CD3ζ (CD3z) domain. This exemplary ligand domain represents the cognate antigen of a HvG disease. Generally, for HLA class II as ligand domain, the ligand domain can consist of the alpha 1 domain and the beta 1 domain, and optionally the alpha 2 and the beta 2 domains, of the HLA class II heavy chains. An example for a signalling molecule according to Fig. 2 is given in SEQ ID NO: 10 with a short linker (also referred to as spacer), wherein the zipper a is embodied by the Jun-Zipper. An example for a signalling molecule according to Fig. 2 with a short linker is given in SEQ ID NO: 11, wherein the zipper b is embodied by the Fos-Zipper as zipper b, which can dimerize with the Jun-Zipper of SEQ ID NO: 10.

A further example for a signalling molecule according to Fig. 2, with a long linker, is given in SEQ ID NO: 12, which includes the Jun-Zipper as a dimerizing domain, and in SEQ ID NO: 13 with a Fos-Zipper, for dimerization with the Jun-Zipper of SEQ ID NO: 12. Fig 3 shows a schematic drawing of an embodiment of the signalling molecule, CLR, of the invention with the transmembrane and signalling domains of two proteins only attached to one of the two heavy chains, here to the alpha 1 and alpha 2 domains of the HLA-DR1 alpha chain HLA-DRA^{∗}01:01. Herein, a first signalling molecule (CLR) consists of the HLA-DR1 alpha chain HLA-DRA^{∗}01:01 as its ligand domain, a linker, a dimerization domain (Zipper), a spacer, a transmembrane domain, and a combination of the intracellular 4-1BB domain and the CD3ζ (CD3z) domain as a signalling domain. A second CLR consisting of a ligand domain represented by beta chain of HLA-DR1 (HLA-DRB1^{∗}01:01:01), a linker and a dimerization domain (Zipper) is bound to the first CLR by the dimerized dimerization domains. An example for a signalling molecule according to Fig. 3 is given in SEQ ID NO: 14, which contains a short linker and wherein the zipper a is embodied by the Jun-Zipper, for dimerization with the Fos-Zipper of SEQ ID NO: 15.

A further example for a signalling molecule according to Fig. 3, with a long linker, is given in SEQ ID NO: 16, which includes the Jun-Zipper as a dimerizing domain, and in SEQ ID NO: 17 with a Fos-Zipper, e.g. for dimerization with the Jun-Zipper of SEQ ID NO: 12.

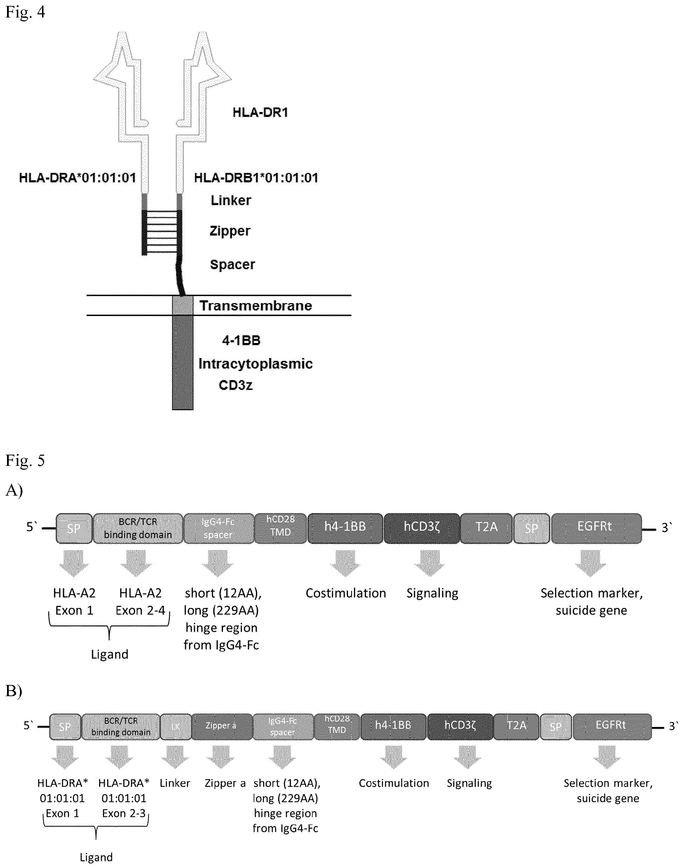

Fig 4 shows a schematic drawing of an embodiment of the signalling molecule, CLR, of the invention with the transmembrane and signalling domains only attached to one of the two heavy chains, here to the beta 1 and beta 2 domains of the HLA-DR1 beta chain. Herein, a first signalling molecule consists of the HLA-DR1 (HLA-DRB1^{∗}01:01:01) as its ligand domain, a linker, a dimerization domain (Zipper), a spacer, a transmembrane domain, and a combination of the intracellular 4-1BB domain and the CD3ζ (CD3z) domain as a signalling domain. A second CLR consisting of a ligand domain represented by beta chain of HLA-DRA^{∗}01:01:01, a linker and a dimerization domain (Zipper) is bound to the first CLR by the dimerized dimerization domains.

An example for a signalling molecule according to Fig. 4 is given in SEQ ID NO: 18, which contains a short linker and wherein the zipper a is embodied by the Fos-Zipper, for dimerization with the Jun-Zipper of SEQ ID NO: 19, also having a short linker.

A further example for a signalling molecule according to Fig. 4, with a long linker, is given in SEQ ID NO: 20, which includes the Fos-Zipper as a dimerizing domain, and in SEQ ID NO: 21 with a Jun-Zipper, e.g. for dimerization with the Fos-Zipper of SEQ ID NO: 20. Generally, for a HLA class II molecule as ligand domain, the ligand domain can consist of the alpha 1 domain and the beta 1 domain, and optionally the alpha 2 and the beta 2 domain, e.g the alpha 1 and alpha 2 domain or the beta 1 and beta 2 domain, of the HLA class II heavy chains, preferably with the extracellular part of the HLA class II transmembrane domain at the C-terminus of the heavy chains.

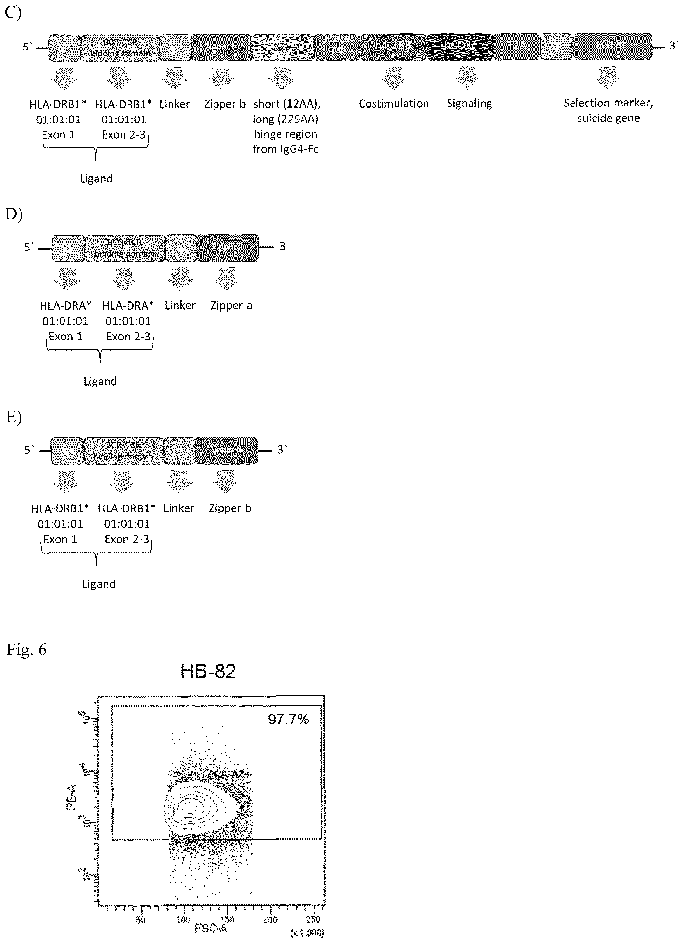

Fig. 5 shows preferred signalling molecules of the invention, in Fig. 5A) a nucleic acid sequence encoding, from 5' to 3', an embodiment of the signalling molecule and as an optional component, separated by a self-cleaving peptide element derived from Thosea asigna Virus 2A (T2A), a selection marker comprising a secretion signal peptide (SP) and a selection marker and/or suicide gene, represented by truncated epidermal growth factor (EGFRt). The encoded signalling molecule contains, from N-terminus to C-terminus, a ligand domain comprised of a secretion signal peptide (SP, exon 1 of HLA-A^{∗}02:01:01) and exons 2-4 of HLA-A^{∗}02:01:01, a spacer (IgG4-Fc spacer), a transmembrane domain (hCD28 TMD) of human CD28, and an intracellular signalling domain of a combination of the co-stimulating h4-1BB domain (h4-1BB) and the hCD3ζ domain (hDC3ζ).

The ligand domain acts as the ligand or binding domain for specific binding to a BCR of a B-cell, resp. of a TCR of a T-cell (BCR/TCR binding domain). As generally preferred, the antigen domain can be a portion of or the entire HLA class I heavy chain sequence. In this example, the antigen domain is the truncated HLA-A2 (A^{∗}02:01:01) heavy chain consisting of the alpha 1, alpha 2 and alpha 3 domains. An exemplary amino acid sequence is given in SEQ ID NO: 1, consisting of an N-terminal signal peptide and the antigen domain consisting of domains alpha 1, alpha 2 and alpha 3 of HLA-A^{∗}02:01.

The spacer can e.g. comprise 10 to 250 amino acids (AA) of a known spacer, e.g. of a Ig hinge region, e.g. 12 AA up to 229 AA of an Ig hinge region, e.g. of the hinge region of IgG4-Fc. Herein, a spacer of 12AA (short) or of 229 AA (long) of the hinge region of IgG4-Fc was used.

In Fig. 5, the preferred extracellular part of a HLA transmembrane domain arranged at the C-terminus of the ligand domain, respectively between the ligand domain and the linker, is not explicitly depicted.

The ligand domain of Fig. 5B) and 5C), which forms the ligand for the TCR, resp. BCR, is comprised of exon 1 of HLA-DRA^{∗}01:01:01 (HLA-DRA^{∗}01:01:01 Exon 1) and exons 2-3 of HLA-DRA^{∗}01:01:01, and exon 1 of HLA-DRB1^{∗}01:01:01 and exon 2-3 of HLA-DRB1^{∗}01:01:01, respectively. The embodiments comprising portions of or entire HLA class II molecules as ligand domains preferably contain a dimerization domain in order to provide for assembly with the HLA class II beta chain of the HLA class II molecule, also referred to as zipping effect.

Fig. 5B) shows an embodiment, in which a dimerization domain is arranged between the ligand domain and the spacer, wherein as preferred the dimerization domain at its N-terminus comprises a linker (Linker). The dimerization domain is shown as one partner of a molecular or leucine zipper (Zipper a) which can be one of the dimerization partners of e.g. a jun/fos zipper, a basic/acidic zipper or a EE1234L/RR1234L zipper. SEQ ID NO: 2 gives the amino acid sequence of a signal peptide, an antigen domain of domains alpha 1 and alpha 2 of HLA-DRA^{∗}01:01, an extracellular part of a transmembrane domain, a linker and Jun zipper (Zipper a) as a dimerization domain.

Fig. 5C) shows an embodiment, in which the dimerization domain is the counterpart of zipper a (Zipper b). SEQ ID NO: 3 gives the amino acid sequence of a signal peptide, a ligand domain of domains beta 1 and beta 2 of HLA-DRB1^{∗}01:01, an extracellular part of a transmembrane domain, a linker and Fos zipper (Zipper b) as a dimerization domain.

Alternative amino acid sequences for a linker domain and a dimerization domain are given in SEQ ID NO: 4 and SEQ ID NO: 5, the zipper portions of which can dimerize, and SEQ ID NO: 6 and SEQ ID NO: 7, the zipper portions of which can dimerize.

### Example: Specific elimination of B-cells

As an example for immune cells causing an undesired immune activity to be treated, the mouse hybridoma B lymphocyte HB-82 cells were used, which express the HLA-A2 specific antibody BB7.2 as a BCR on their cell surface and in culture secrete the HLA-A2 specific antibody BB7.2 into the supernatant. Fig. 6 shows a FACS result of detecting the BB7.2 antibody on the surface of HB-82 cells stained with anti-mouse IgG (F(ab)₂), labelled with Phycoerythrin. This shows that BB7.2 is not only secreted in the supernatant but also strongly expressed on the surface of HB-82 cells.

The antibody produced in the supernatant of HB-82 cells was capable of immunologically staining HLA-A2+ lymphocytes in FACS analysis using as a second antibody anti-mouse IgG (F(ab)₂), labelled with Phycoerythrin as well as killing HLA-A2+ lymphocytes in complement dependent cytotoxicity assays. This shows that the BB7.2 antibody binds to the HLA-A2+ lymphocytes.

In a separate culture, CD8+ T-cells were stimulated by anti-CD3 and anti-CD38 antibodies for controlled expansion. As a control, K562 cells were treated in the same manner. On the next day, the cells were lentivirally transduced with a nucleic acid sequence encoding a CLR according to Fig. 5A), comprising the exons 2 to 4 of HLA-A^{∗}02:01:01 as the ligand domain. In parallel batches, two variants of the CLR were encoded by the lentiviral vector, namely one having a short spacer of 12 AA (SEQ ID NO: 8), and one having a long spacer of 229 AA (SEQ ID NO: 9) of the hinge region of IgG4-Fc. On day 9 after stimulation, the transduced T-cells and K562 control cells were sorted by flow cytometry (FACS) using the co-expressed EGFRt as a selection marker.

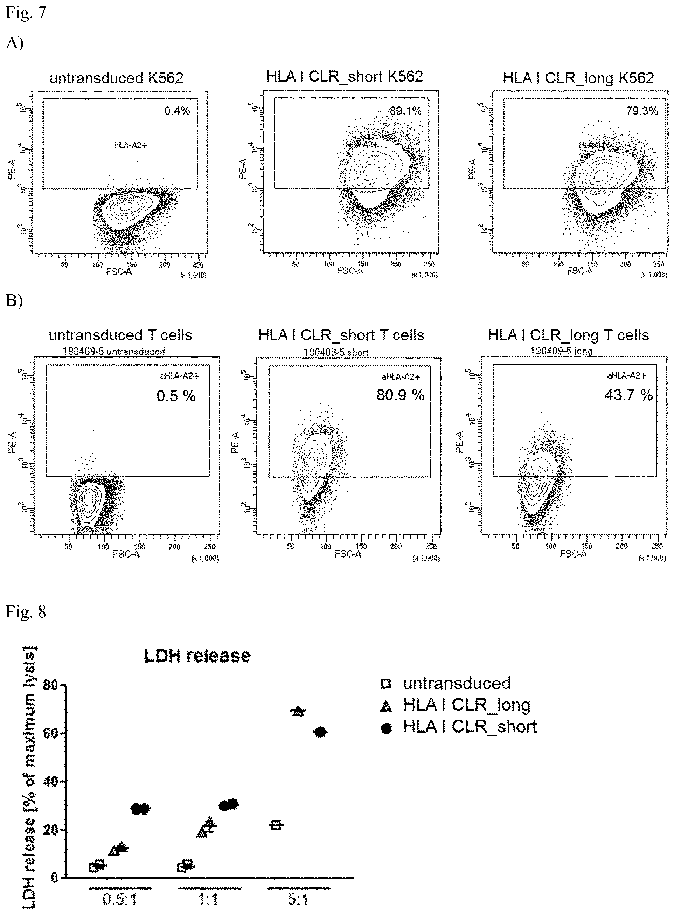

Cells expressing the selection marker were cultivated for a further 5 d and analysed for expression of the CLR and for their activity against immune cells directed against the ligand domain of the CLR. The transduced cells were contacted with the supernatant of HB-82 cells containing the BB7.2 antibody. Secondary staining was done by anti-mouse IgG (F(ab)2), labelled with Phycoerythrin. The FACS results are depicted in Fig. 7, showing that the BB7.2 antibody effectively binds to the immune cells expressing the CLR, specifically binding to the ligand domain of the CLR.

Fig. 7 shows FACS results, in A) for control cells, and in B) for T-cells, namely for untransduced T-cells (untransduced T cells) and untransduced control cells (untransduced K562), for cells transduced with the construct containing the short spacer T-cells (HLA I CLR_short T cells) or control cells (HLA I CLR_short K562), and for cells transduced with the construct containing the long spacer T-cells (HLA I CLR_long T cells) or control cells (HLA I CLR_long K562). The results show that the CLR is strongly expressed both in control cells (K562), and in T-cells, wherein the short spacer showed a stronger expression of the CLR, in particular in T cells.

The activity of T-cells expressing the CLR was analysed by contacting and co-cultivating them with freshly washed mouse hybridoma B lymphocyte HB-82, which express the HLA-A2 specific BCR BB7.2, which correlates to the secreted antibody BB7.2. The cells expressing the CLR were used at a ratio of 0.5:1 or 1:1, or 5:1 with HB-82 cells. For all ratios, an effective killing of the HB-82 cells was observed, showing that the expression of the CLR by T-cells results in effective elimination of the B-cells that express a BCR specific for the ligand domain of the CLR. For the CLR having the short spacer, a higher cytotoxic effectivity of the T-cell expressing it was determined. The results of the analysis of lactate dehydrogenase (LDH) released after 48 h of co-cultivation is depicted in Fig. 8 showing that in all ratios for T-cells expressing the CLR an effective cytotoxicity was found.

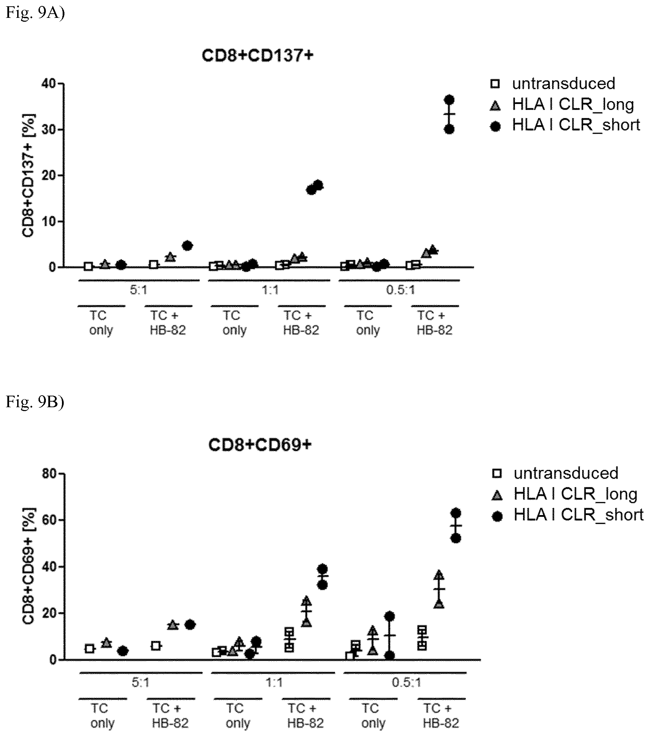

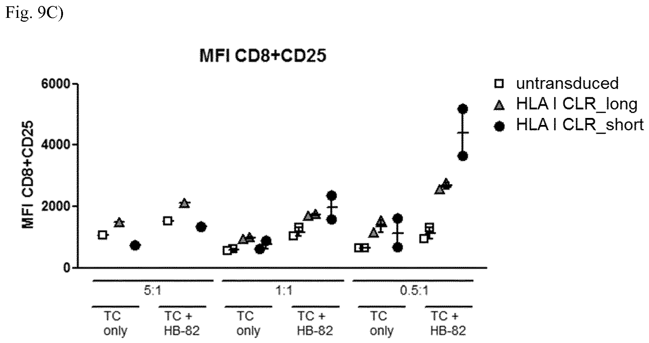

In addition, the expression of activation markers on T-cells expressing the CLR was analysed after 48 h of co-cultivation at effector-to-target ratios of 5:1 or 1:1, and 0.5:1. The results for CD8+ T-cells are shown in Fig. 9 A) for CD137, in Fig. 9B) for CD69, and in Fig. 9C) for CD25. These results show that the CD8+ T-cells that were transduced to express the CLR clearly showed expression of the activation markers CD137, CD69, and CD25, for all ratios and both for the short and for the long spacer variants, with a stronger activation for the short spacer variant.

These results on the example of a B-cell receptor show that the signalling molecule CLR of the invention directs T-cells that express this signalling molecule to kill immune cells, exemplified by B-cells, with specificity for the cognate antigen against which the immune cells are directed, e.g. with specificity for the B-cell receptor, resp. T-cell receptor, of the immune cells.

## Claims

1. Fusion protein for use as a signalling molecule, comprising a ligand domain, a spacer, a transmembrane domain, and an intracellular signalling domain, wherein the ligand domain contains at least one epitope of a cognate antigen, against which an undesired immune response is directed.

2. Fusion protein according to claim 1, comprising a dimerization domain arranged between the ligand domain and the transmembrane domain.

3. Fusion protein according to one of the preceding claims, wherein the spacer has a length of 10 to 250 amino acids.

4. Fusion protein according to one of the preceding claims, wherein the intracellular signalling domain is a combination of a h4-1BB domain and a hCD3ζ domain, or a combination of an intracellular hCD28 signalling domain and a hCD3ζ domain.

5. Fusion protein according to one of the preceding claims, wherein the ligand domain comprises at least a portion of a HLA class I or at least a portion of a HLA class II molecule.

6. Fusion protein according to claim 5, wherein the portion of the HLA class I contains at least one mutation in amino acid position No. 74, 223, 224, 225, 226, 227, 229, and 245, wherein the numbering refers to HLA class I without signal peptide.

7. Fusion protein according to claim 5 or 6, wherein the portion of the HLA class II contains at least one mutation in amino acid position No. 88, 90 and 176 in the alpha chain, and/or a mutation in at least one amino acid position of Nos. 46, 54, 55, 56, 104, 114, 116, 134, 135, 136, 137, 138, 139, 141, 142, 143, 144, 145, 148, 158, 160, and 162 in the beta chain, wherein the numbering refers to HLA class II without signal peptide.

8. Fusion protein according to one of the preceding claims, comprising a first signalling molecule comprising a ligand domain, optionally an extracellular part of a HLA transmembrane domain, a linker, a dimerization domain, a spacer, a transmembrane domain, and at least one intracellular signalling domain, wherein the dimerization domain is dimerized with the dimerization domain of a second signalling molecule, the second signalling molecule comprising a ligand domain, an extracellular part of a HLA transmembrane domain, a linker, a dimerization domain, a spacer, a transmembrane domain, and at least one intracellular signalling domain, or the second signalling molecule consisting of a ligand domain, a linker and a dimerization domain.

9. Fusion protein according to one of the preceding claims, comprising a first signalling molecule comprising or consisting of a ligand domain, optionally an extracellular part of a HLA transmembrane domain, a spacer, a transmembrane domain, and at least one intracellular signalling domain.

10. Fusion protein according to one of the preceding claims for use in the treatment of immune rejections against transplants, for use in the treatment of autoimmune diseases, or for use in the treatment of allergies.

11. Fusion protein according to claim 10 for use in the treatment of autoimmune diseases, wherein the ligand domain is the cognate antigen against which the autoimmune disease is directed.

12. Immune cell expressing a fusion protein according to one of the preceding claims for use in the treatment of immune rejections against transplants, for use in the treatment of autoimmune diseases, or for use in the treatment of allergies.

13. Immune cell according to claim 12, wherein the immune cell is a T-cell, a primary T-cell, a NK cell, or a progenitor cell of one of these or a cell line.

14. Immune cell according to one of claims 12 to 13, wherein the immune cell is immunologically compatible with the recipient.

15. Immune cell according to one of claims 12 to 14, wherein the immune cell is for administration to a patient prior to or following transplantation.

16. Nucleic acid sequence encoding a fusion protein according to one of claims 1 to 11.
